# EUROPEAN PATENT APPLICATION

(11) **EP 4 682 906 A1**
(43) Date of publication of application: **21.01.2026**
(21) Application number: 24771018.9
(22) Date of filing: 15.03.2024
(51) Int. Cl.: G16H 50/50, A61B 10/00, G16H 50/20

(54) **INFORMATION PROCESSING DEVICE, INFORMATION PROCESSING METHOD, PROGRAM, AND STORAGE MEDIUM**

(30) Priority: 16.03.2023 JP 2023042430
(71) Applicant: Eisai R&D Management Co., Ltd., Tokyo 112-8088 (JP)
(72) Inventor: AOSHIMA, Ken, Tsukuba-shi, Ibaraki 300-2635 (JP); NAKAMURA, Yoshitaka, Tokyo 112-8088 (JP); MIURA, Yuji, Tokyo 112-8088 (JP); ANABUKI, Kenichi, Tokyo 112-8088 (JP); DAIRIKI, Ryo, Tokyo 112-8088 (JP); TSUNEYOSHI, Momoka, Tokyo 112-8088 (JP); SEGAWA, Emiko, Tokyo 112-8088 (JP); NAGAOKA, Kazuya, Tokyo 112-8088 (JP)
(74) Representative: Hoffmann Eitle
(86) International application number: PCT/JP2024/010360
(87) International publication number: WO 2024/190916

(57) **Abstract**

Patient information corresponding to each of at least three variables is input to a prediction model trained using training data in which the three variables are associated with presence or absence of progression of symptoms of mild cognitive impairment (MCI) or mild Alzheimer's dementia (mild AD) to predict a probability of progression of patient's symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers, wherein, with respect to at least four pieces of information, selected from the first information, the second information, the third information, and the fourth information and including: at least one of information on orientation relating to time from among the first information and information on remembering a holiday, a family gathering, an appointment, or a medication schedule in IADL from among the third information; information on delayed recall from among the second information; and information on positivity and negativity of amyloid β from among the fourth information, the at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

## Description

### Technical Field

The present invention relates to an information processing device, an information processing method, a program, and a storage medium.

### Background Art

Conventionally, various technologies have been proposed to predict the degree of dementia symptoms. For example, Patent Literature 1 discloses predicting the severity of dementia by analyzing free conversations of a patient without performing Mini Mental State Examination (MMSE) which is used for screening and assessing the severity of dementia.

### Citation List

### Patent Literature

Patent Literature 1: Patent Publication JP-A-2020-42659

### Summary of Invention

### Technical Problem

In recent years, in order to improve the therapeutic effect on dementia patients, in addition to predicting dementia, it has been required to predict the disease in patients with mild cognitive impairment (MCI) or mild Alzheimer's dementia (mild AD), who are potential dementia patients. However, since the symptoms of mild cognitive impairment (MCI) or mild Alzheimer's dementia (mild AD) patients are milder than those of dementia patients, it has been difficult to accurately predict the disease.

The present invention has been made in view of such circumstances, and an object of the present invention is to provide an information processing device, an information processing method, a program, and a storage medium capable of accurately predicting the probability of progression of symptoms of mild cognitive impairment (MCI) or mild Alzheimer's dementia (mild AD).

### Solution to Problem

The present disclosure provides an information processing device, wherein patient information corresponding to each of at least three variables is input to a prediction model trained using training data in which the three variables are associated with presence or absence of progression of symptoms of mild cognitive impairment (MCI) or mild Alzheimer's dementia (mild AD) to predict a probability of progression of a patient's symptoms of mild cognitive impairment (MCI) or mild Alzheimer's dementia (mild AD), the at least three variables being based on information selected from among first information on orientation, second information on remembering, third information on remembering a holidays, a family gathering, an appointment, or a medication schedule in IADL or an actual action of eating, shopping, or traveling in IADL, and fourth information on positivity and negativity biomarkers, wherein, with respect to at least four pieces of information, selected from the first information, the second information, the third information, and the fourth information and including: at least one of information on orientation relating to time from among the first information and information on remembering a holiday, a family gathering, an appointment, or a medication schedules in IADL from among the third information; information on delayed recall from among the second information; and information on positivity and negativity of amyloid β (Aβ) from among the fourth information, the at least three variables include at least one of a first feature amount obtained by integrating information in which magnitudes of evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

The at least three variables may include at least one of the first feature amount and the second feature amount with respect to at least four pieces of information including: information on remembering a holiday, a family gathering, an appointment, or a medication schedule in IADL from among the third information; information on delayed recall from among the second information; and information on positivity and negativity of Aβ from among the fourth information.

The at least three variables may include at least one of the first feature amount and the second feature amount with respect to at least four pieces of information including: information on orientation relating to time from among the first information; information on remembering a holiday, a family gathering, an appointment, or a medication schedule in IADL from among the third information; information on delayed recall from among the second information; and information on positivity and negativity of Aβ from among the fourth information.

The at least three variables may include at least one of the first feature amount and the second feature amount with respect to four or five pieces of information.

The at least three variables may include at least one of the first feature amount and the second feature amount with respect to four pieces of information including: information on orientation relating to time from among the first information, information on remembering a holiday, a family gathering, an appointment, or a medication schedule in IADL from among the third information; information on delayed recall from among the second information; and information on positivity and negativity of Aβ from among the fourth information.

The at least three variables may include at least one of the first feature amount and the second feature amount with respect to five pieces of information including: information on orientation to time from among the first information, information on remembering a holiday, a family gathering, an appointment, or a medication schedule in IADL from among the third information; information on delayed recall from among the second information; information on positivity and negativity of Aβ from among the fourth information; and fifth information selected from the first information, the second information, the third information, and the fourth information.

The at least three variables may include at least one of the first feature amount and the second feature amount with respect to at least four pieces of information, selected from the first information, the second information, the third information, and the fourth information and including at least one of information on orientation relating to time from among the first information and information on remembering a holiday, a family gathering, an appointment, or a medication schedule in IADL from among the third information, information on delayed recall from among the second information, and information on positivity and negativity of Aβ from among the fourth information.

The present disclosure provides an information providing method comprising the steps of: acquiring patient information; predicting a probability of progression of patient's symptoms of mild cognitive impairment (MCI) or mild Alzheimer's dementia (mild AD) by inputting patient information corresponding to each of at least three variables to a prediction model trained using training data in which the three variables are associated with presence or absence of progression of symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers; and outputting a prediction result on the probability of progression of patient's symptoms of mild cognitive impairment (MCI) or mild Alzheimer's dementia (mild AD), wherein, with respect to at least four pieces of information, selected from the first information, the second information, the third information, and the fourth information and including at least one of information on orientation relating to time from among the first information and information on remembering a holiday, a family gathering, an appointment, or a medication schedule in IADL from among the third information; information on delayed recall from among the second information; and information on positivity and negativity of Aβ from among the fourth information, the at least three variables include at least one of a first feature amount obtained by integrating information in which magnitudes of evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

The present disclosure provides a program causing a computer to execute: processing of acquiring patient information; processing of predicting a probability of progression of patient's symptoms of mild cognitive impairment (MCI) or mild Alzheimer's dementia (mild AD) by inputting patient information corresponding to each of at least three variables to a prediction model trained using training data in which the three variables are associated with presence or absence of progression of symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers; and processing of outputting a prediction result on the probability of progression of patient's symptoms of mild cognitive impairment (MCI) or mild Alzheimer's dementia (mild AD), wherein, with respect to at least four pieces of information, selected from the first information, the second information, the third information, and the fourth information and including: at least one of information on orientation relating to time from among the first information, and information on remembering a holiday, a family gathering, an appointment, or a medication schedule in IADL from among the third information; information on delayed recall from among the second information; and information on positivity and negativity of Aβ from among the fourth information, the at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

The present disclosure provides a non-transitory computer-readable storage medium storing the aforementioned program.

### Advantageous Effect of Invention

According to the present invention, the probability of progression of symptoms of mild cognitive impairment (MCI) or mild Alzheimer's dementia (mild AD) can be accurately predicted.

### Brief Description of Drawings

[Figure 1] Figure 1 is a block diagram showing an example of a configuration of an information processing device according to the present embodiment.
[Figure 2] Figure 2 is a diagram for describing an example of a learning phase of a prediction model.
[Figure 3] Figure 3 is a diagram for describing an example of an evaluation phase of the prediction model.
[Figure 4] Figure 4 is a diagram for describing an example of an evaluation index of the prediction model.
[Figure 5] Figure 5 is a diagram for describing an example of an execution phase of the prediction model.
[Figure 6] Figure 6 is a flowchart showing an example of a process of predicting the probability of progression of patient's symptoms of mild cognitive impairment (MCI) or mild Alzheimer's dementia (mild AD).
[Figure 7] Figure 7 is a graph showing an example of change in the degree of progression of symptoms of mild cognitive impairment (MCI) or mild Alzheimer's dementia (mild AD) over time.
[Figure 8] Figure 8 is a diagram for describing an evaluation method for MMSE.
[Figure 9] Figure 9 is a diagram for describing an evaluation method for FAQ.
[Figure 10] Figure 10 is a diagram for describing an evaluation method for ADAS.
[Figure 11] Figure 11 is a diagram for describing the contents of IADL.
[Figure 12] Figure 12 is a diagram for describing an example of attribute information of training data.
[Figure 13] Figure 13 is a diagram for describing the operation of the information processing device of the present embodiment.
[Figure 14] Figure 14 is a diagram showing an example of data used to evaluate a prediction model of an example.
[Figure 15] Figure 15 is a diagram showing an example of data used to evaluate a prediction model of an example.
[Figure 16] Figure 16 is a diagram showing an example of data used to evaluate a prediction model of an example.
[Figure 17] Figure 17 is a diagram showing an example of data used to evaluate a prediction model of an example.
[Figure 18] Figure 18 is a diagram showing an example of data used to evaluate a prediction model of an example.
[Figure 19] Figure 19 is a diagram showing an example of data used to evaluate a prediction model of an example.
[Figure 20] Figure 20 is a diagram showing an example of data used to evaluate a prediction model of an example.
[Figure 21] Figure 21 is a diagram showing an example of data used to evaluate a prediction model of an example.
[Figure 22] Figure 22 is a diagram showing an example of data used to evaluate a prediction model of an example.
[Figure 23] Figure 23 is a diagram showing an example of data used to evaluate a prediction model of an example.
[Figure 24] Figure 24 is a diagram showing an example of data used to evaluate a prediction model of an example.
[Figure 25] Figure 25 is a diagram showing an example of data used to evaluate a prediction model of an example.
[Figure 26] Figure 26 is a diagram showing an example of data used to evaluate a prediction model of an example.
[Figure 27] Figure 27 is a diagram showing an example of data used to evaluate a prediction model of an example.
[Figure 28] Figure 28 is a diagram showing an example of data used to evaluate a prediction model of an example.
[Figure 29] Figure 29 is a diagram showing an example of data used to evaluate a prediction model of an example.
[Figure 30] Figure 30 is a diagram showing an example of data used to evaluate a prediction model of an example.
[Figure 31] Figure 31 is a diagram showing an example of data used to evaluate a prediction model of an example.

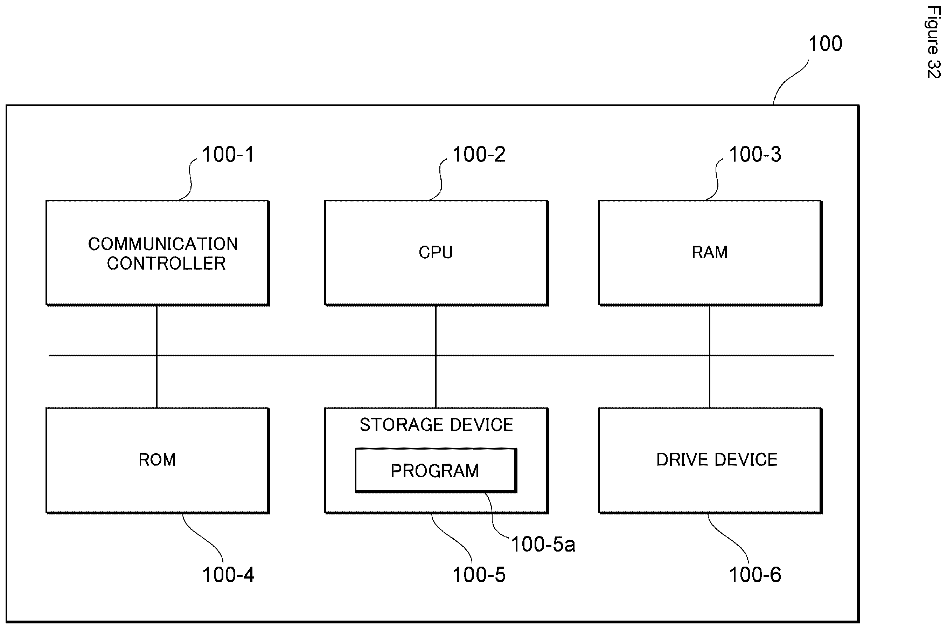

[Figure 32] Figure 32 is a diagram showing an example of a hardware configuration of the information providing device according to the present embodiment.

### Description of Embodiments

Hereinafter, an embodiment of an information processing device will be described with reference to the drawings. The present disclosure is not limited to the embodiment described below.

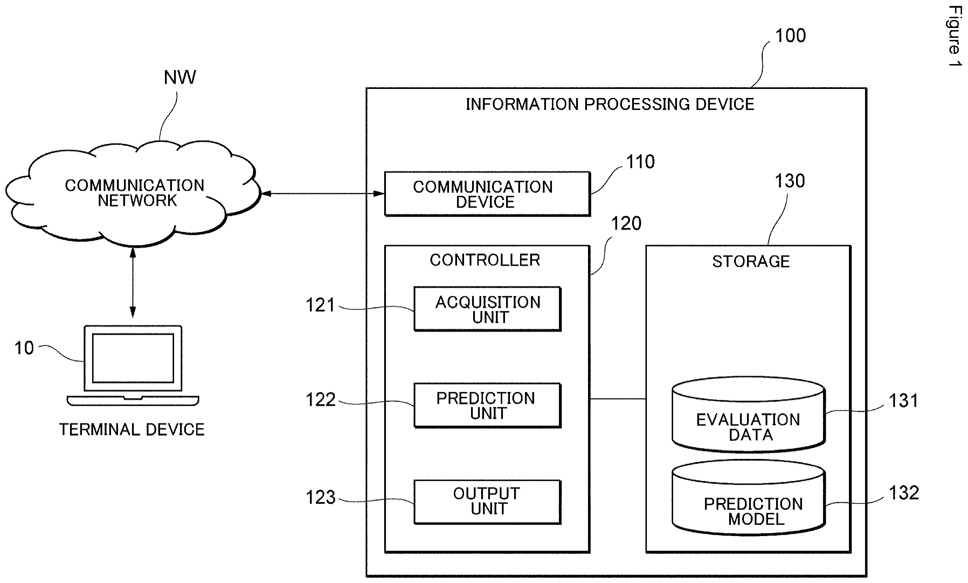

As shown in Figure 1, the information processing device 100 is connected to, for example, a terminal device 10 via a communication network NW. The communication network NW is composed of, for example, a communication device, a wireless communication network, etc.

The terminal device 10 is a device to which a doctor inputs patient information, and is configured as, for example, a computer terminal device or a mobile information terminal device.

The information processing device 100 includes, for example, a communication device 110, a controller 120, and a storage 130. The controller 120 is implemented by, for example, a hardware processor such as a central processing unit (CPU) executing a program (software). In addition, some or all of such components may be implemented by hardware (including circuitry) such as a large scale integration (LSI) circuit, an application specific integrated circuit (ASIC), a field-programmable gate array (FPGA), a graphics processing unit (GPU), etc., or may be implemented by software and hardware in cooperation. The program may be stored in advance in the storage 130 of the information processing device 100, or may be stored in a removable computer-readable recording medium such as a DVD or a CD-ROM, and may be installed in the storage 130 of the information processing device 100 by setting the computer-readable recording medium in a drive device.

The communication device 110 includes a communication interface such as a network interface card (NIC). The communication device 110 communicates with the terminal device 10, for example, using a cellular network or a Wi-Fi (registered trademark) network.

The controller 120 includes, for example, an acquisition unit 121, a prediction unit 122, and an output unit 123.

The acquisition unit 121 acquires patient information transmitted from the terminal device 10 through the communication network NW. Here, the patient information is information used at the time of predicting the probability of progression of patient's symptoms of mild cognitive impairment (MCI) or mild Alzheimer's dementia (mild AD). Details of the patient information will be described later.

The prediction unit 122 inputs the patient information acquired by the acquisition unit 121 to a prediction model 132 and predicts the probability of progression of patient's symptoms of mild cognitive impairment (MCI) or mild Alzheimer's dementia (mild AD).

The output unit 123 outputs information on the probability of progression of patient's symptoms of mild cognitive impairment (MCI) or mild Alzheimer's dementia (mild AD) predicted by the prediction unit 122 to the terminal device 10 through the communication device 110. For example, in response to a request from the patient, the output unit 123 may output the information on the probability of progression of patient's symptoms of mild cognitive impairment (MCI) or mild Alzheimer's dementia (mild AD) predicted by the prediction unit 122 to a medical professional such as a doctor or a terminal device carried by the patient.

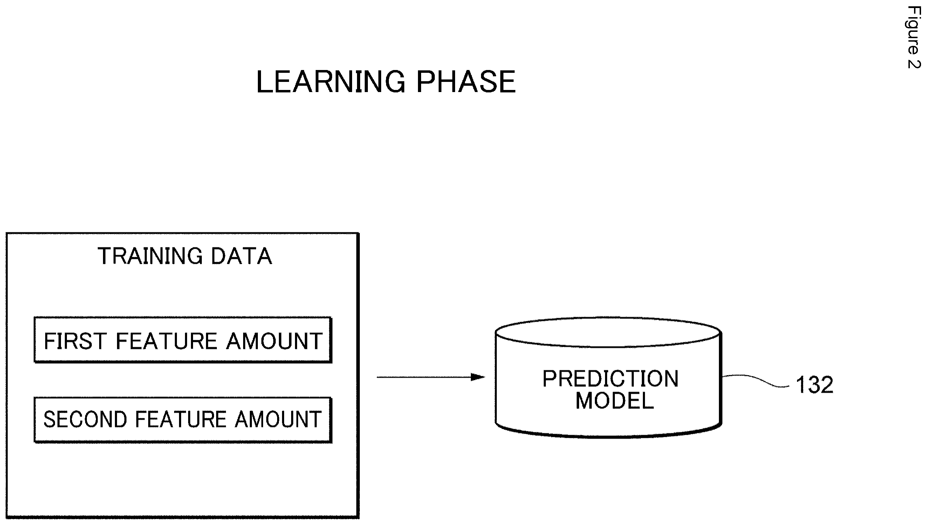

Figure 2 is a diagram showing an example of a learning phase of the prediction model 132.

In the example shown in Figure 2, the prediction model 132 is trained using data in which attribute information of subjects as a first data group is associated with diagnostic information on mild cognitive impairment (MCI) or mild Alzheimer's dementia (mild AD) of the subjects as a second data group and which is extracted from among cohort data (ADNI) which will be described below, for each of a plurality of subjects. In the present embodiment, as will be described later, as an example of attribute information of a subject, when information on orientation is defined as first information, information on remembering is defined as second information, information on remembering holidays, family gatherings, appointments, or medication schedules in IADL, or information on actual actions of eating, shopping, or traveling in IADL is defined as third information, and information on positivity and negativity of biomarkers is defined as fourth information, it is preferable that at least four pieces of information selected from the first information, the second information, the third information, and the fourth information, including at least one of information on orientation to time among the first information, and information on remembering holidays, family gatherings, appointments, or medication schedules in IADL from among the third information, information on delayed recall from among the second information, and information on positivity and negativity of Aβ from among the fourth information include at least one of a first feature amount obtained by integrating information in which magnitudes of evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

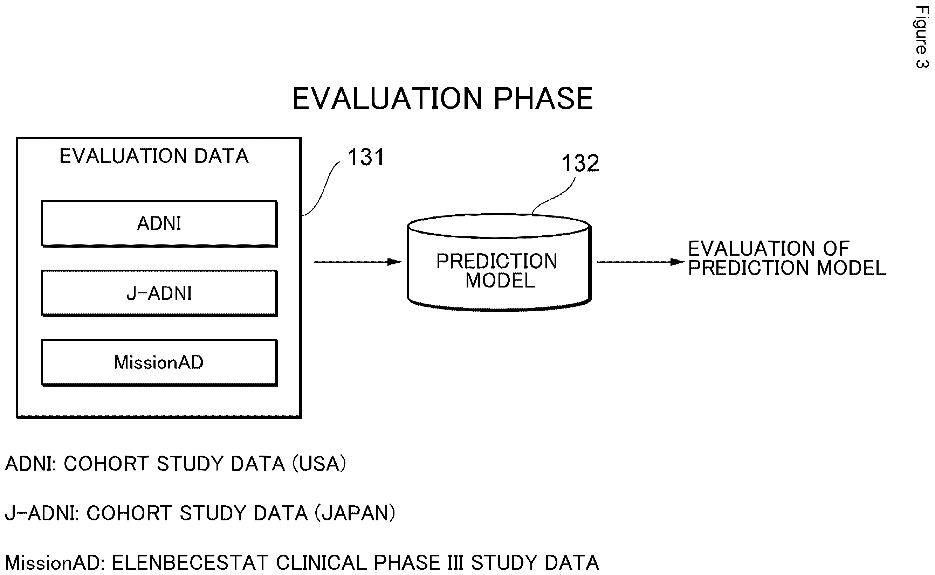

Figure 3 is a diagram showing an example of an evaluation phase of the prediction model 132.

In the example shown in Figure 3, the prediction model 132 trained as described above is evaluated using evaluation data 131. In the present embodiment, for the evaluation data 131, three databases, ADNI (Alzheimer's Disease Neuroimaging Initiative), J-ADNI (Japanese Alzheimer's Disease Neuroimaging Initiative), and MissionAD (elenbecestat clinical phase III study data), are used, and the prediction model 132 is evaluated individually for each database. ADNI is a database based on cohort data collected for a plurality of clinical periods in order to advance clinical trials of dementia disease modifying drugs in the United States. J-ADNI is a database based on cohort data collected for a plurality of clinical periods to advance clinical trials of dementia disease modifying drugs in Japan. MissionAD is a database based on clinical trial data independently collected by the applicant to advance clinical trials of dementia disease modifying drugs. Since the databases have different sets of subjects, evaluation of the prediction model 132 using these databases is meaningful in determining the range of patients to which the prediction model 132 is applicable.

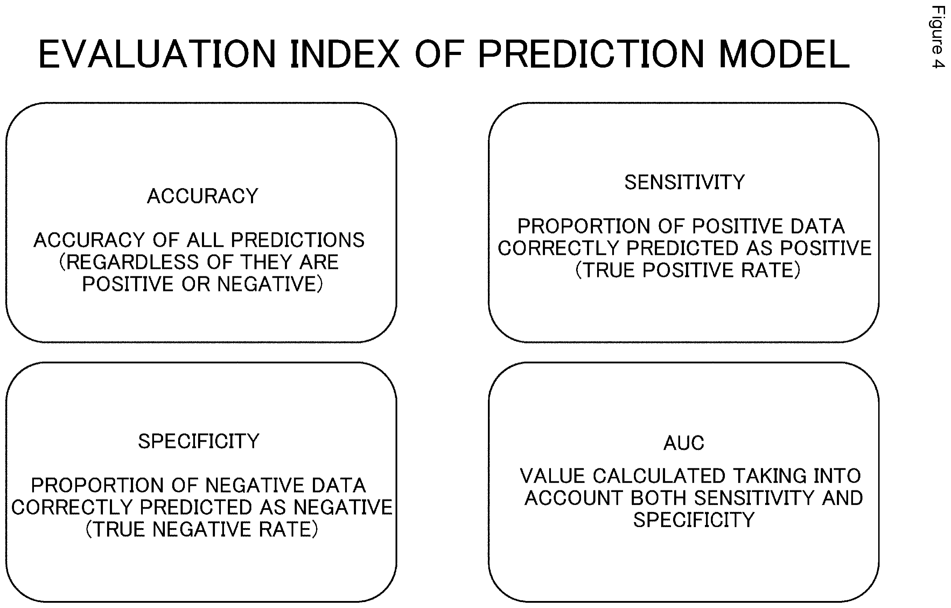

Figure 4 is a diagram for describing an example of an evaluation index of the prediction model 132.

In the example shown in Figure 4, accuracy, sensitivity, specificity, and AUC (Area Under the ROC Curve) are provided as examples of the evaluation index of the prediction model 132. The accuracy is an index indicating the overall accuracy regardless of whether it is positive or negative. The sensitivity is an index indicating the proportion of positive data correctly predicted as positive (true positive rate). The specificity is an index indicating the proportion of negative data correctly predicted as negative (true negative rate). The AUC is an index calculated taking into account both the sensitivity and the specificity. In general, when evaluating the prediction model 132 as a whole, evaluation based on AUC is preferable, and in one embodiment, in addition to AUC, sensitivity is added, and in another embodiment, sensitivity and specificity are added. In yet another embodiment, accuracy, sensitivity, and specificity are added. For each index, the criterion for determining whether the prediction result is suitable/unsuitable is 0.7, 0.67, or 0.6 or more.

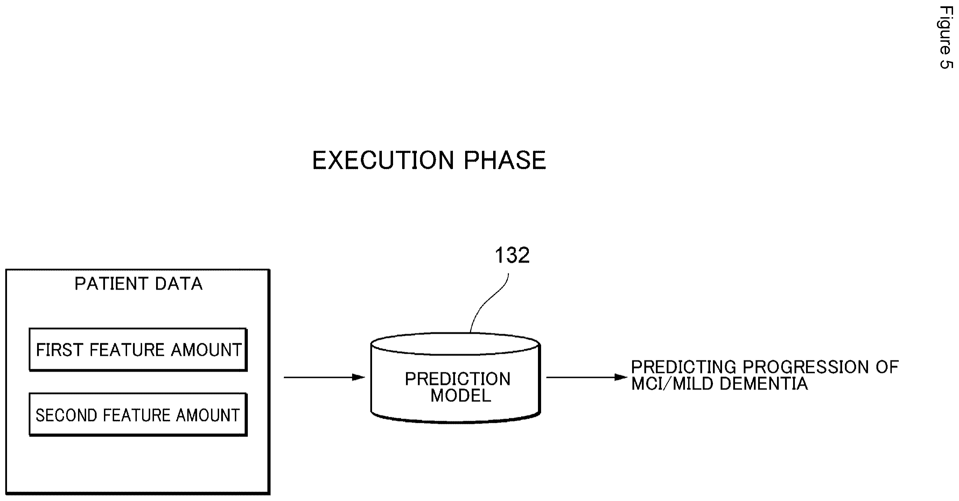

In the example shown in Figure 5, patient information acquired from the terminal device 10 is input to the prediction model 132 trained as described above, and the probability of progression of patient's symptoms of mild cognitive impairment (MCI) or mild Alzheimer's dementia (mild AD) is predicted. In the present embodiment, as will be described later, it is preferable that at least one of the first feature amount and the second feature amount described above be included as an example of attribute information of patient information.

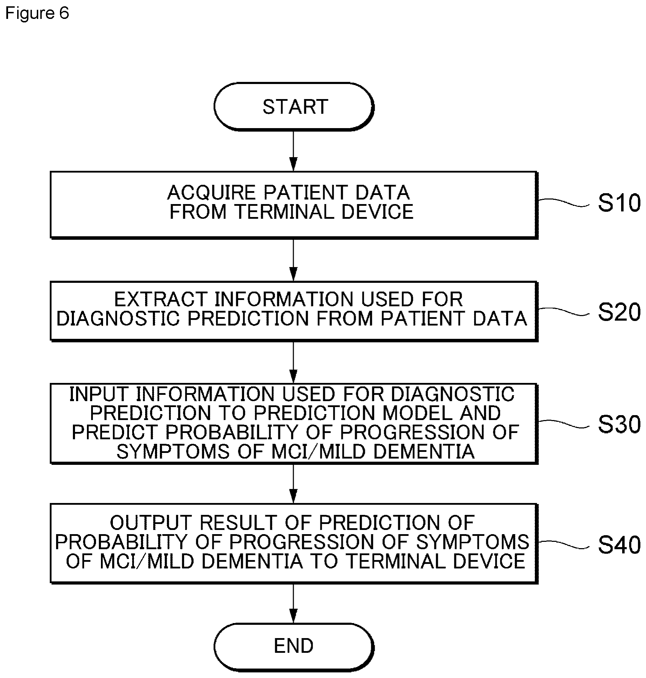

As shown in Figure 6, the information processing device 100 first acquires patient data from the terminal device 10 (step S10).

Next, the information processing device 100 extracts information used for diagnostic prediction from the patient data acquired in the previous step S10 (step S20).

Next, the information processing device 100 inputs the information used for diagnostic prediction extracted in the previous step S20 to the prediction model 132, and predicts the probability of progression of patient's symptoms of mild cognitive impairment (MCI) or mild Alzheimer's dementia (mild AD) (step S30).

Next, the information processing device 100 outputs a prediction result on the probability of progression of patient's symptoms of mild cognitive impairment (MCI) or mild Alzheimer's dementia (mild AD) in the previous step S30 to the terminal device 10 (step S40), and ends the flowchart shown in Figure 6.

In the present embodiment, patient information is information obtained by, for example, a doctor interviewing a patient for medical examination. Examples of methods of interviewing for medical examination include Mini-Mental State Examination (MMSE), Functional activities questionnaire (FAQ), and Alzheimer's Disease Assessment Scale (ADAS) (Dementia Disease Management Guidelines 2017 (https://www.neurology-jp.org/guidelinem/nintisyo_2017.html), Japanese Journal of Geriatrics 2011;48:431-438 (refer to https://jpn-geriat-soc.or.jp/publications/other/pdf/review_geriatrics_48_5_431.pdf).

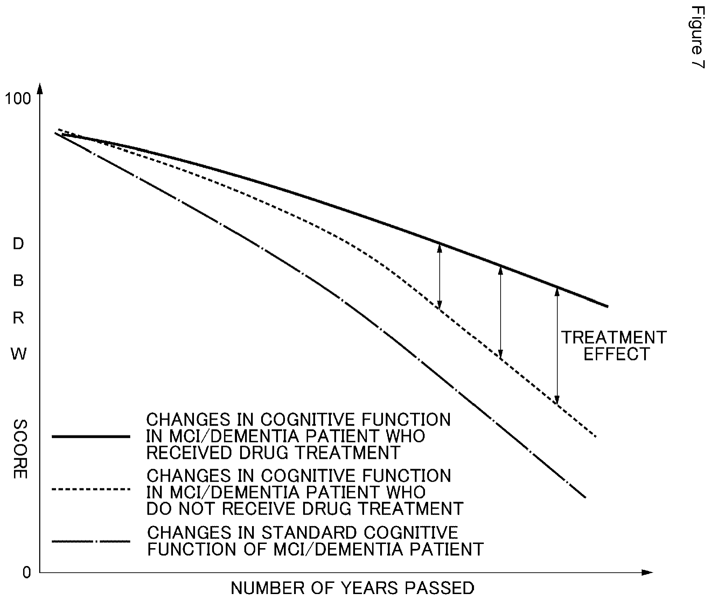

Figure 7 is a graph showing an example of change in the degree of progression of symptoms of mild cognitive impairment (MCI) or mild Alzheimer's dementia (mild AD) over time predicted by the prediction model 132. In the example shown in the figure, onset prediction (symptom progression prediction) of mild cognitive impairment (MCI) or mild Alzheimer's dementia (mild AD) at a future point in time is performed over time using the prediction model 132. In this case, based on time series data of cohort data (ADNI) from the past to the present time, each prediction model 132 that predicts onset prediction (symptom progression prediction) of mild cognitive impairment (MCI) or mild Alzheimer's dementia (mild AD) at each future point in time, such as six months later, one year later, two years later, five years later, and ten years later, is generated, for example. Then, by inputting cohort data (ADNI) at the present time to such a plurality of prediction models 132, it is possible to continuously plot the degree of progression of symptoms of mild cognitive impairment (MCI) or mild Alzheimer's dementia (mild AD). Then, by referring to the graph shown in this example, it is also possible to compare and predict changes in degrees of progression of symptoms over time and the effects of drug treatment between cases in which a patient with mild cognitive impairment (MCI) or mild Alzheimer's dementia (mild AD) receives drug treatment and cases in which the patient does not receive drug treatment.

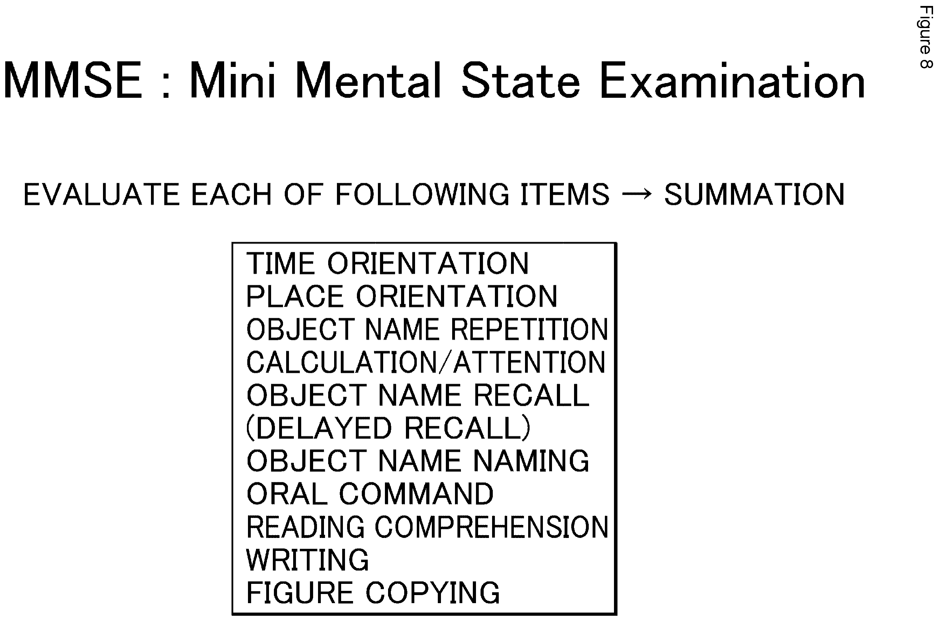

As shown in Figure 8, MMSE is a type of neuropsychological test performed when dementia is suspected. In MMSE, dementia is diagnosed by scoring a plurality of diagnostic items and summing the scores, and the lower the total score, the more advanced symptoms are. MMSE diagnostic items include, for example, time orientation, place orientation, object name repetition, attention, object name recall (delayed recall), object name naming, sentence repetition, three-step oral command, reading comprehension, writing, and figure copying.

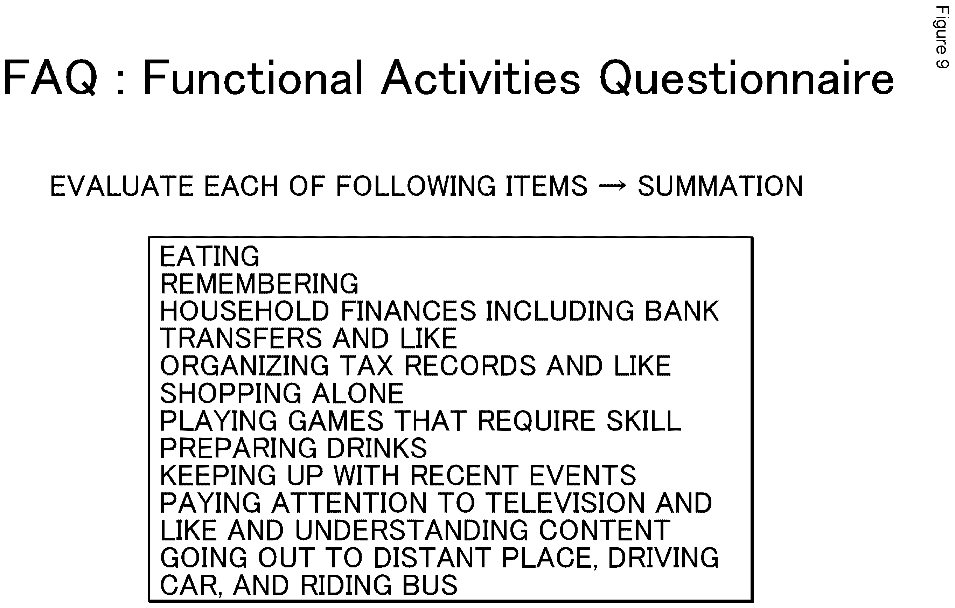

As shown in Figure 9, FAQ is a method of objectively evaluating the life management ability of a dementia patient. In FAQ, dementia is diagnosed by scoring a plurality of diagnostic items and summing the scores, and a higher total score indicates a lower life management ability. The plurality of diagnostic items are used to evaluate abilities related to, for example, eating, remembering, household management, organization, shopping, games, preparing drinks, keeping up with recent events, understanding what is being shown on television, and traveling.

### <Question Items>

· Remembering appointments, family gatherings, holidays, and taking medications (herein referred to as FAQ-evaluated remembering or simply FAQ remembering)
· Preparing a balanced meal (herein referred to as FAQ-evaluated meal or simply FAQ meal)
· Going out to a distant place, driving a car, and riding a bus: travel (herein referred to as FAQ-evaluated travel or simply FAQ travel)
· Shopping alone: shopping (herein referred to as FAQ-evaluated shopping or simply FAQ shopping)
· Household management, including bank transfers
· Organizing tax records, etc.
· Playing games that require skill
· Preparing drinks
· Keeping up with recent events
· Paying attention to television, etc. and understanding the content

### <Score>

0: Can do without difficulty
1: Can do on their own with some difficulty
2: Requires help
3: Full assistance

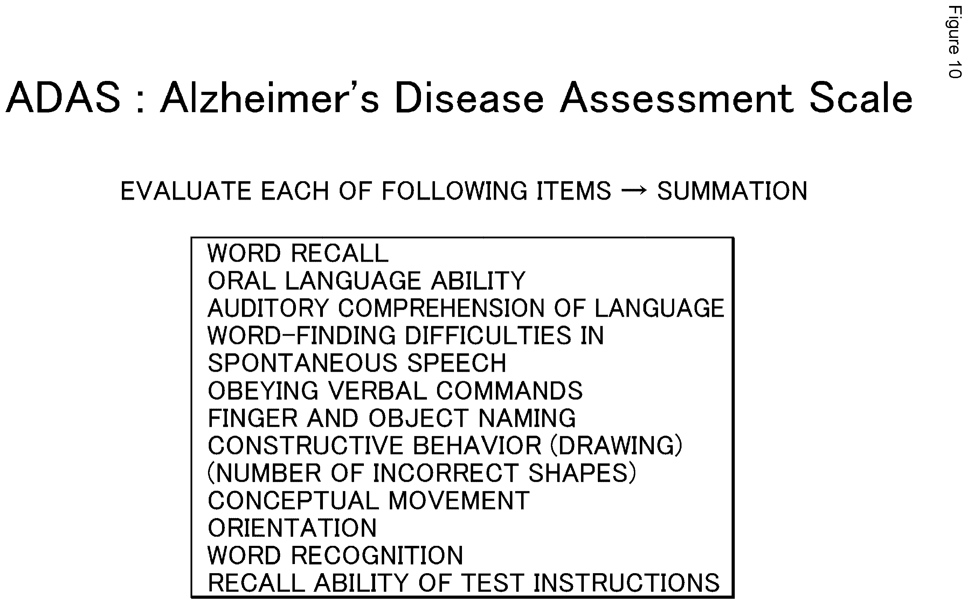

As shown in Figure 10, ADAS is a test that is performed a plurality of times continuously to evaluate changes in cognitive function based on changes in scores. ADAS scores a plurality of diagnostic items, with higher scores indicating more advanced symptoms. The plurality of diagnostic items are used to evaluate abilities related to word recall, oral language ability, auditory comprehension of language, word-finding difficulties in spontaneous speech, comprehension of oral commands, finger and object naming, constructive behavior, conceptual movement, orientation, word recognition, and recall of test instructions.

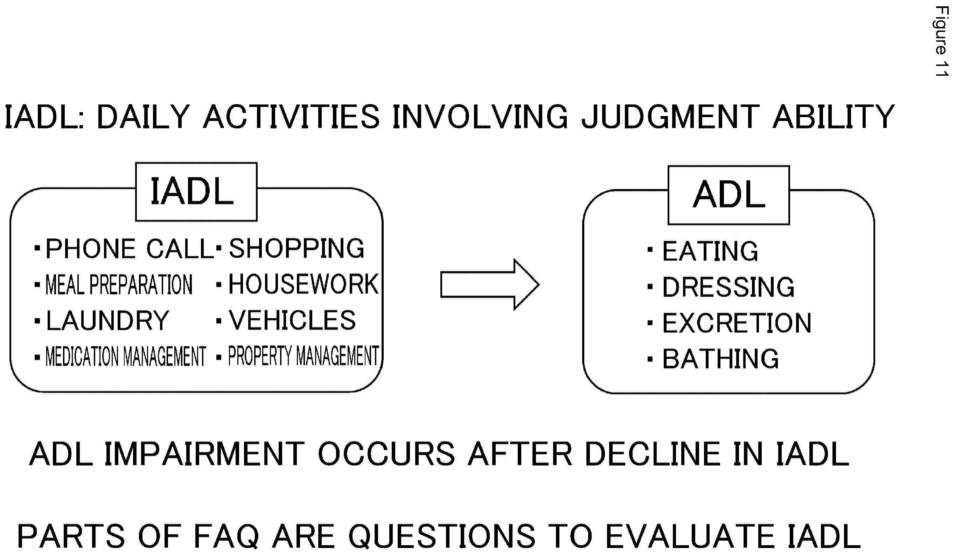

As shown in Figure 11, in the present embodiment, patient information includes, for example, information on orientation, information on remembering, information on Instrumental Activities of Daily Living (IADL) (refer to (https://www.mhlw.go.jp/www1/topics/kenko21_11/s1.html)), and information on positivity and negativity of amyloid β.

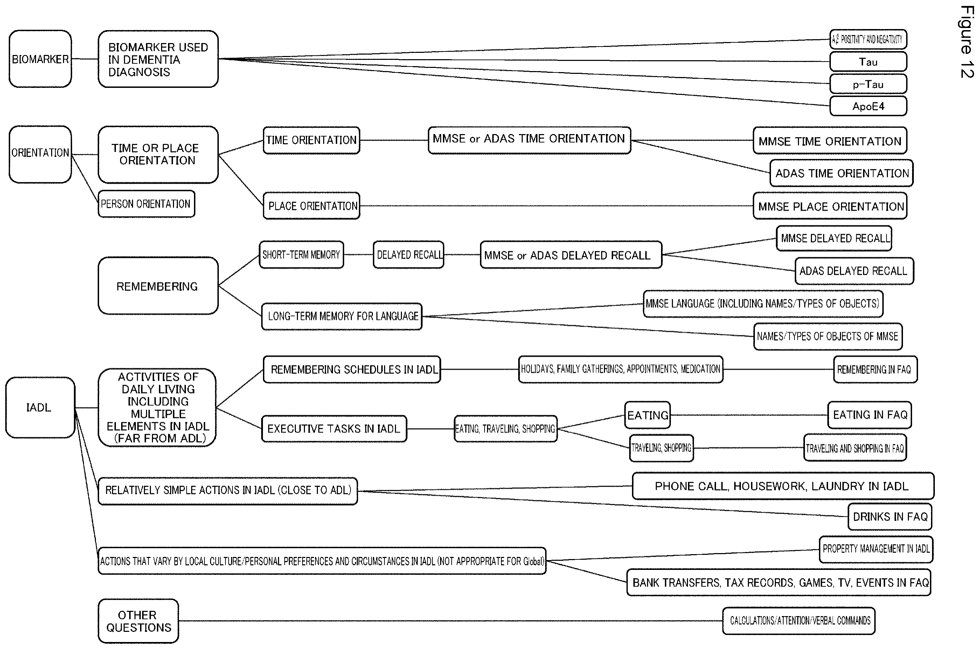

Figure 12 is a diagram for describing an example of attribute information of data used for evaluation of the prediction model 132, showing branching from higher-level concepts to lower-level concepts from left to right on the page. As shown in Figure 11, the information on orientation is information on the ability to comprehensively determine the date, current time, location, surrounding situation, understanding of people, etc., and to ascertain and understand the situation in which a patient is currently placed, and includes, for example, information on time orientation, information on place orientation, and information on person orientation. The information on time orientation is, for example, information indicating a score obtained from the patient with respect to items related to time orientation in MMSE or ADAS. The information on place orientation is, for example, information indicating a score obtained from the patient with respect to items related to place orientation in the MMSE.

The information on remembering is information on the ability of a patient to retain short-term or long-term memory, and includes, for example, information on delayed recall and information on long-term language memory. The information on delayed recall is information on the ability to recall memorized matters after a certain period of time has passed. The information on delayed recall is, for example, information indicating a score obtained from the patient with respect to delayed recall items in MMSE or ADAS. The information on delayed recall includes a question item of object name repetition. The information on long-term language memory is information on the ability to memorize language matters for a long period of time, and includes, for example, information on the ability of a subject to repeat three words immediately after a tester conveyed to the subject these three words at one-second intervals (object name repetition), information on the ability to name the correct name of an object while looking at the object prepared by the tester (object naming), and information on the ability to accurately remember the contents conveyed by the tester (delayed recall). The information on long-term language memory is, for example, information indicating a score obtained from the patient with respect to an item such as object name naming in MMSE.

Information on IADL is information on daily activities involving judgment ability, for example, information for numerically evaluating abilities related to phone call, meal preparation, laundry, medication management, shopping, housework, vehicles, and property management. In general, since there is a decline in evaluation values for ADL (Activities of Daily Living) such as eating, dressing, excretion, and bathing, which are indicators of dementia after a decline in evaluation values with respect to IADL has been observed, information on IADL is useful for identifying early signs of dementia. Information on IADL is classified based on whether it is activities of daily living including a plurality of elements, relatively simple activities of daily living, or activities that differ due to local culture, personal preferences, and circumstances. The activities of daily living including a plurality of elements are activities far from ADL, and are classified into activities related to the ability to remember schedules in IADL, such as holidays, family gatherings, appointments, and medication management, and activities related to executive tasks in IADL, such as meal preparation, travel, and shopping. Information on remembering related to schedules in IADL is, for example, information indicating scores of answers given by a patient with respect to items such as holidays, family gatherings, appointments, and medication management in FAQ. Information on executive tasks in IADL is, for example, information indicating scores given by a patient with respect to items such as meal preparation and travel in FAQ. Relatively simple activities of daily living are activities close to ADL, and are activities related to telephone, housework, laundry, and abilities related to housework. Information on relatively simple activities of daily living in IADL is, for example, information indicating scores given by a patient with respect to telephone, housework, laundry, and the like. In addition, activities that differ due to local culture and personal preferences, and circumstances correspond to abilities related to property management and games that require skill, for example. Information on activities that differ due to local culture and personal preferences, and circumstances in IADL is, for example, information indicating scores obtained from a patient in neuropsychological tests (including FAQ, etc.) with respect to property management and games that require skill.

A biomarker is an item/substance in a living body that is an indicator of the presence or absence of a disease, changes in the condition, or the effect of treatment. Items used as biomarkers are mainly data derived from living bodies, such as blood pressure, heart rate, electrocardiogram, and substances such as proteins measured in the blood. Information on positivity and negativity of biomarker may be information on evaluation and judgment obtained from Tau, p-Tau, ApoE4, etc. in addition to information on evaluation and judgment obtained from amyloid β. Tau is a protein that accumulates in the brain in various neurodegenerative diseases including Alzheimer's disease, causing the death of neurons and causing dementia. p-Tau means phosphorylated tau protein, and the amount of accumulation correlates with the severity of dementia in an abnormal structure that appears in the brain of Alzheimer's disease patients. ApoE4 is apolipoprotein E (ApoE) gene subtype ε4 (ApoE-ε4), and is attracting attention as a genetic factor closely related to AD. Information on positivity and negativity of biomarkers is information indicating whether a certain amount of a biomarker, which is a factor highly correlated with the onset of dementia, has accumulated in the brain of a patient, and is information obtained, for example, through imaging diagnosis or blood diagnosis (including CSF tests and the like). When a biomarker is positive, it suggests that disease progression of dementia is progressing, compared to when the biomarker is negative.

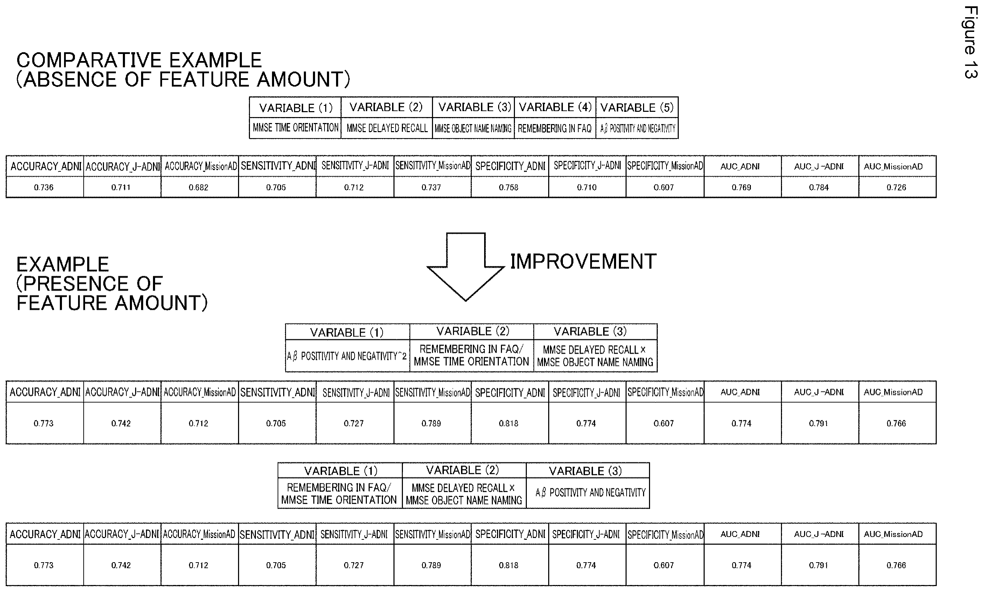

Next, the operation of the information processing device 100 of the present embodiment will be described with reference to Figure 13 based on evaluation indices of a prediction model 132. In the example shown in Figure 13, a prediction model 132 is given as a comparative example, in which five pieces of information are variables, the variables being information on time orientation evaluated by MMSE, information on delayed recall evaluated by MMSE, information on remembering object name naming evaluated by MMSE, information on remembering holidays, family gatherings, appointments, or medication schedules in IADL evaluated by FAQ, and information on Aβ positivity and negativity.

In this example, similarly, two prediction models 132 including the above-mentioned first feature amount and second feature amount as variables are given as examples, which are based on five pieces of information consisting of five pieces of information on time orientation evaluated by MMSE, information on delayed recall evaluated by MMSE, information on remembering object name naming evaluated by MMSE, information on remembering holidays, family gatherings, appointments, or medication schedules in IADL evaluated by FAQ, and information on Aβ positivity and negativity.

When a feature amount is generated using information on delayed recall evaluated by MMSE and information on language memory evaluated by MMSE, the magnitudes of evaluation values indicating degrees of symptoms of mild cognitive impairment (MCI) or mild Alzheimer's dementia (mild AD) show a positive correlation, and thus the first feature amount obtained by integrating the information on delayed recall evaluated by MMSE and the information on language memory evaluated by MMSE is used as a variable.

A lower total score indicates a more advanced symptom in MMSE, and a higher total score indicates a poorer ability to manage living in FAQ. That is, in MMSE and FAQ, the magnitudes of evaluation values indicating degrees of symptoms of mild cognitive impairment (MCI) or mild Alzheimer's dementia (mild AD) show a negative correlation. Therefore, as in the prediction model 132 of the above-mentioned example, when a feature amount is generated using information on remembering holidays, family gatherings, appointments, or medication schedules in IADL evaluated by FAQ and information on time orientation evaluated by MMSE, the second feature amount obtained by dividing the information on remembering holidays, family gatherings, appointments, or medication schedules in IADL evaluated by FAQ by information on time orientation evaluated by MMSE is used as a variable.

Examples of evaluation indices of the prediction model 132 include accuracy, sensitivity, specificity, and AUC (Area Under the ROC Curve), and evaluation values for these four evaluation indices are calculated using evaluation data of ADNI, J-ADNI, and MissionAD. In the example shown in Figure 12, the prediction model 132 of the example is improved in all of the four evaluation indices compared to the prediction model 132 of the comparative example, and therefore it can be said that the performance is superior.

Next, an example of the prediction model 132 of the present embodiment will be described with reference to the drawings. As described above, examples of evaluation indices of the prediction model 132 include accuracy, sensitivity, specificity, and AUC (Area Under the ROC Curve). The method of using the evaluation indices of the prediction model 132 is not particularly limited, but in the present embodiment below, as an example, in evaluation of the prediction model 132 using ADNI, J-ADNI, and MissionAD, a prediction model 132 that satisfies the conditions of "sensitivity ≥ 0.7," "specificity ≥ 0.6," and "AUC ≥ 0.7" is used as an example. Here, the example of the present disclosure described below is one aspect, and is not limited to the example described below.

### (Conditions for Implementation)

In construction and evaluation of the prediction model, first, a model for predicting presence or absence of symptom progression using 80% of ADNI data, in which various pieces of information are associated with presence or absence of symptom progression, as training data is constructed. The remaining 20% of the ADNI data, J-ADNI and MissionAD data, are then used as evaluation data, and presence or absence of symptom progression based on the prediction model is compared with actual presence or absence of symptom progression to calculate various evaluation indices.

### (Example)

Data used to evaluate the prediction model 132 of the example is data that was not used to construct the prediction model 132 from among the training data, and includes at least three variables based on information selected from first information on orientation, second information on remembering, third information on remembering holidays, family gatherings, appointments, or medication schedules in IADL, or actual actions of eating, shopping, or traveling in IADL, and fourth information on positivity and negativity of biomarkers. The at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation, with respect to at least four pieces of information selected from the first information, the second information, the third information, and the fourth information, which include at least one of information on time orientation from among the first information, and information on remembering holidays, family gatherings, appointments, or medication schedules in IADL from among the third information, information on delayed recall from among the second information, and information on positivity and negativity of amyloid β from among the fourth information. The at least three variables may include the first feature amount and the second feature amount calculated by integrating or dividing a plurality of pieces of information with respect to at least four pieces of information including information other than the first information, the second information, the third information, and the fourth information.

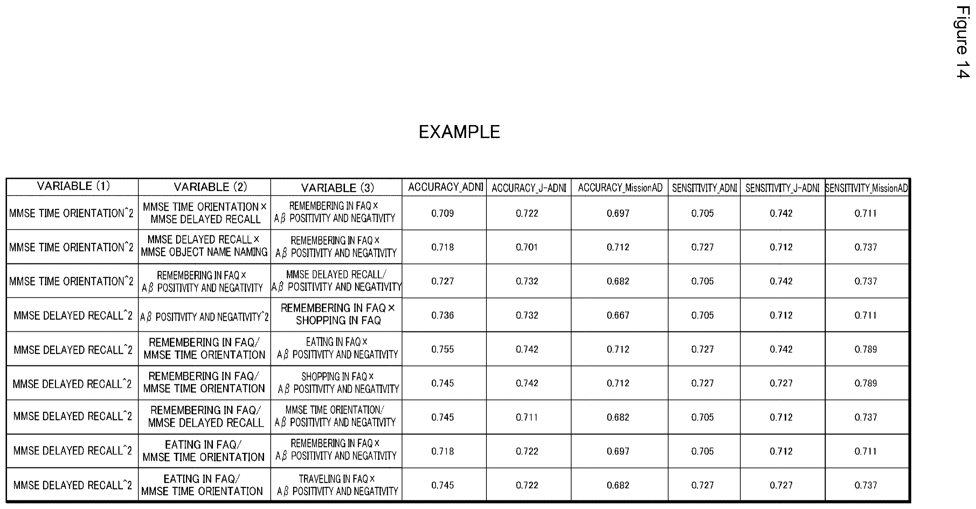

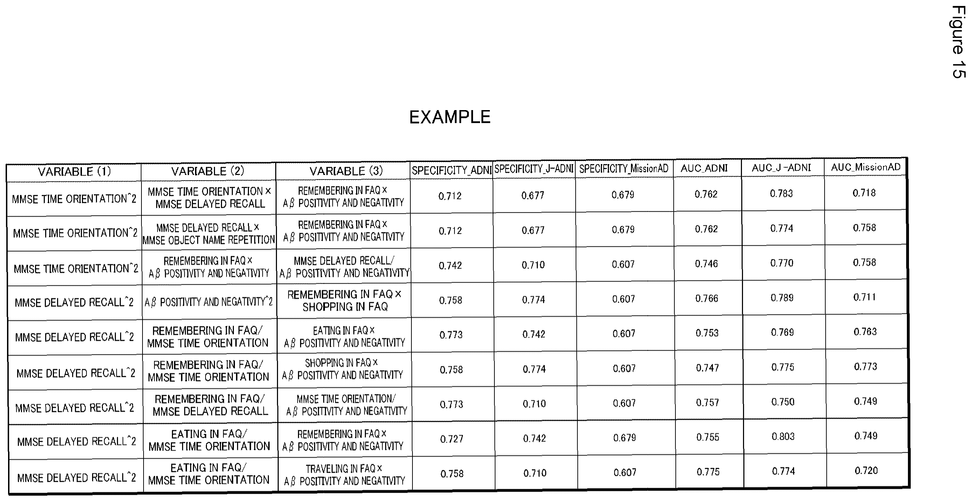

Figures 14 and 15 are diagrams showing examples of data used to evaluate prediction models 132 of the example. Figure 14 shows an example of evaluation values for accuracy and sensitivity, which are examples of evaluation indices of prediction models 132, using evaluation data of ADNI, J-ADNI, and MissionAD. Figure 15 shows an example of evaluation values for specificity and AUC, which are examples of evaluation indices of the prediction models 132, using evaluation data of ADNI, J-ADNI, and MissionAD. In the examples shown in Figures 14 and 15, in the data used to evaluate the prediction models 132, for example, the at least three variables include a first variable that is information obtained by squaring information on time orientation from among the first information, a second variable that includes, as a first feature amount, a feature amount obtained by integrating the information on time orientation from among the first information and information on delayed recall from among the second information, and a third variable that includes, as a first feature amount, a feature amount obtained by integrating information on remembering holidays, family gatherings, appointments, or medication schedules in IADL evaluated by FAQ from among the third information, and information on positivity and negativity of amyloid β from among the fourth information. In addition, the at least three variables include, for example, a first variable that is information obtained by squaring information on time orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on remembering holidays, family gatherings, appointments, or medication schedules in IADL evaluated by FAQ from among the third information and information on positivity and negativity of amyloid β from among the fourth information. Furthermore, the at least three variables include, for example, a first variable that is information obtained by squaring information on time orientation from among the first information, a second variable that includes, as a first feature amount, a feature amount obtained by integrating information on remembering holidays, family gatherings, appointments, or medication schedules in IADL evaluated by FAQ from among the third information and information on positivity and negativity of amyloid β from among the fourth information, and a third variable that includes, as a second feature amount, a feature amount obtained by dividing information on delayed recall from among the second information by information on positivity and negativity of amyloid β from among the fourth information. In addition, the at least three variables include, for example, a first variable that is information obtained by squaring information on delayed recall from among the second information, a second variable that is information obtained by squaring information on positivity and negativity of amyloid β from among the fourth information, and a third variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information. Furthermore, the at least three variables include, for example, a first variable that is information obtained by squaring information on delayed recall from among the second information, a second variable that includes, as a second feature amount, a feature amount obtained by dividing information on remembering holidays, family gatherings, appointments, or medication schedules in IADL evaluated by FAQ from among the third information by information on time orientation from among the first information, and a third variable that includes, as a first feature amount, a feature amount obtained by integrating information on eating in IADL evaluated by FAQ from among the third information and information on positivity and negativity of amyloid β from among the fourth information. Furthermore, the at least three variables include, for example, a first variable that is information obtained by squaring information on delayed recall from among the second information, a second variable that includes, as a second feature amount, a feature amount obtained by dividing information on remembering holidays, family gatherings, appointments, or medication schedules in IADL evaluated by FAQ from among the third information by information on time orientation from among the first information, and a third variable that includes, as a first feature amount, a feature amount obtained by integrating information on shopping evaluated by FAQ from among the third information and information on positivity and negativity of amyloid β from among the fourth information. In addition, the at least three variables include, for example, a first variable that is information obtained by squaring information on delayed recall from among the second information, a second variable that includes, as a second feature amount, a feature amount obtained by dividing information on remembering holidays, family gatherings, appointments, or medication schedules in IADL evaluated by FAQ from among the third information by information on delayed recall from among the second information, and a third variable that includes, as a second feature amount, a feature amount obtained by dividing information on time orientation from among the first information by information on positivity and negativity of amyloid β from among the fourth information. Furthermore, the at least three variables include, for example, a first variable that is information obtained by squaring information on delayed recall from among the second information, a second variable that includes, as a second feature amount, a feature amount obtained by dividing information on eating in IADL evaluated by FAQ from among the third information by information on time orientation from among the first information, and a third variable that includes, as a first feature amount, a feature amount obtained by integrating information on remembering holidays, family gatherings, appointments, or medication schedules in IADL evaluated by FAQ from among the third information and information on positivity and negativity of amyloid β from among the fourth information. Furthermore, the at least three variables include a first variable that is information obtained by squaring information on delayed recall from among the second information, a second variable that includes, as a second feature amount, a feature amount obtained by dividing information on eating in IADL evaluated by FAQ from among the third information by information on time orientation from among the first information, and a third variable that includes, as a first feature amount, a feature amount obtained by integrating information on travel in IADL evaluated by FAQ from among the third information and information on positivity and negativity of amyloid β from among the fourth information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

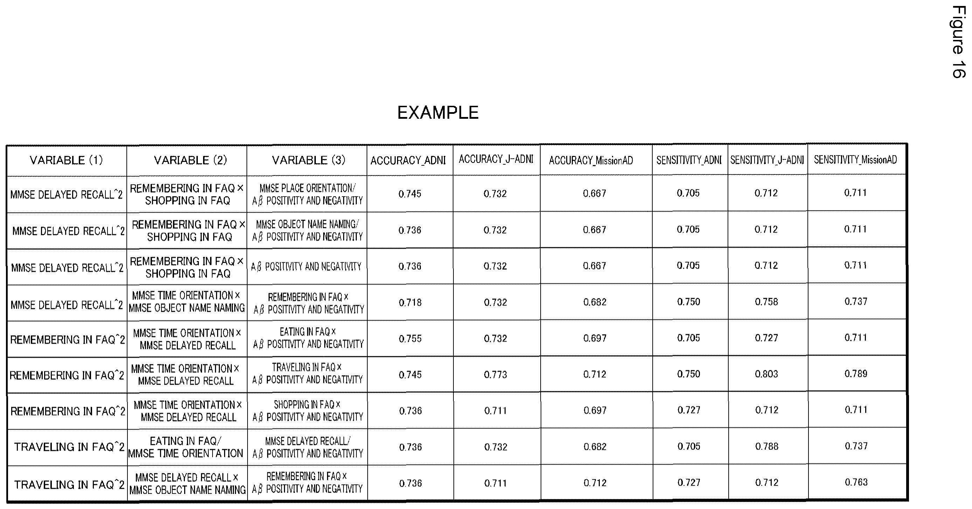

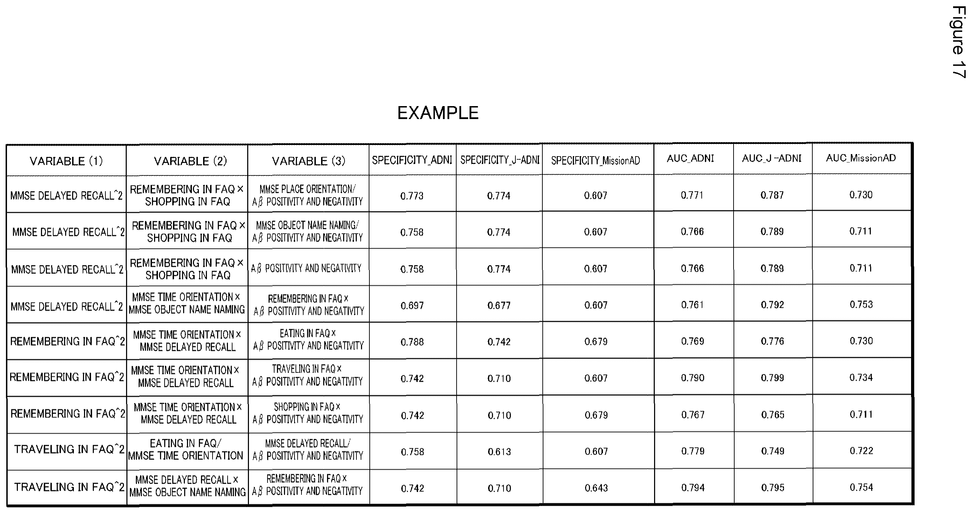

Figures 16 and 17 are diagrams showing examples of data used to evaluate the prediction models 132 of the example. Figure 16 shows an example of evaluation values for accuracy and sensitivity, which are examples of evaluation indices of the prediction models 132, using the evaluation data of ADNI, J-ADNI, and MissionAD. Figure 17 shows an example of evaluation values for specificity and AUC, which are examples of evaluation indices of the prediction models 132, using the evaluation data of ADNI, J-ADNI, and MissionAD. In the examples shown in Figures 16 and 17, in the data used to evaluate the prediction models 132, for example, the at least three variables include a first variable that is information obtained by squaring information on delayed recall from among the second information, a second variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, and a third variable which includes, as a second feature amount, a feature amount obtained by dividing information on place orientation from among the first information by information on the positivity and negativity of amyloid β from among the fourth information. Furthermore, the at least three variables include, for example, a first variable that is information obtained by squaring information on delayed recall from among the second information, a second variable that includes, as a first feature, a feature amount obtained by integrating information on remembering holidays, family gatherings, appointments, or medication schedules in IADL evaluated by FAQ from among the third information and information on shopping in IADL evaluated by FAQ from among the third information, and a third variable that includes, as a second feature, a feature amount obtained by dividing information on object name naming from among the second information by information on positivity and negativity of amyloid β from among the fourth information. In addition, the at least three variables include, for example, a first variable which is information obtained by squaring information on delayed recall from among the second information, a second variable which includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, and a third variable which is information on the positivity and negativity of amyloid β from among the fourth information. Further, the at least three variables include a first variable which is information obtained by squaring information on delayed recall from among the second information, a second variable which includes, as a first feature amount, a feature amount obtained by integrating information on time orientation from among the first information and information on object name naming from among the second information, and a third variable which includes, as a first feature amount, a feature amount obtained by integrating information on remembering holidays, family gatherings, appointments, or medication schedules in IADL evaluated by FAQ from among the third information and information on positivity and negativity of amyloid β from among the fourth information. In addition, the at least three variables include, for example, a first variable which is information obtained by squaring information on remembering holidays, family gatherings, appointments, or medication schedules in IADL evaluated by FAQ from among the third information, a second variable which includes, as a first feature amount, a feature amount obtained by integrating information on time orientation from among the first information and information on delayed recall from among the second information, and a third variable which includes, as a first feature amount, a feature amount obtained by integrating information on eating in IADL evaluated by FAQ from among the third information and information on the positivity and negativity of amyloid β from among the fourth information. Furthermore, the at least three variables include, for example, a first variable which is information obtained by squaring information on remembering holidays, family gatherings, appointments, or medication schedules in IADL evaluated by FAQ from among the third information, a second variable which includes, as a first feature amount, a feature amount obtained by integrating information on time orientation from among the first information and information on delayed recall from among the second information, and a third variable which includes, as a first feature amount, a feature amount obtained by integrating information on travel in IADL evaluated by FAQ from among the third information and information on positivity and negativity of amyloid β from among the fourth information. In addition, the at least three variables include a first variable which is information obtained by squaring information on remembering holidays, family gatherings, appointments, or medication schedules in IADL evaluated by FAQ from among the third information, a second variable which includes, as a first feature amount, a feature amount obtained by integrating information on time orientation from among the first information and information on delayed recall from among the second information, and a third variable which includes, as a first feature amount, a feature amount obtained by integrating information on shopping in IADL evaluated by FAQ from among the third information and information on the positivity and negativity of amyloid β from among the fourth information. Furthermore, the at least three variables include, for example, a first variable that is information obtained by squaring information on travel in IADL evaluated by FAQ from among the third information, a second variable that includes, as a second feature amount, a feature amount obtained by dividing information on eating in IADL evaluated by FAQ from among the third information by information on time orientation from among the first information, and a third variable that includes, as a second feature amount, a feature amount obtained by dividing information on delayed recall from among the second information by information on the positivity and negativity of amyloid β from among the fourth information. Furthermore, the at least three variables include, for example, a first variable which is information obtained by squaring information on travel in IADL evaluated by FAQ from among the third information, a second variable which includes, as a first feature amount, a feature amount obtained by integrating information on delayed recall from among the second information and information on object name naming from among the second information, and a third variable which includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

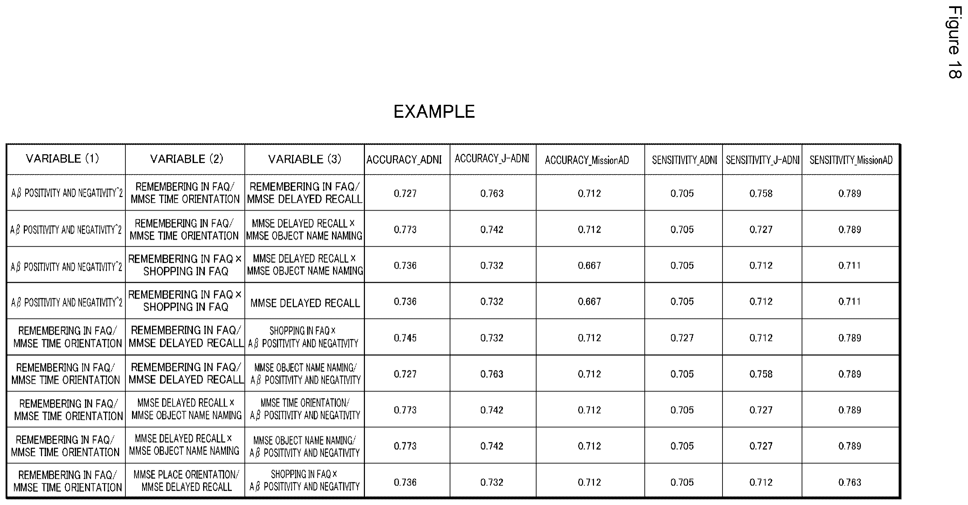

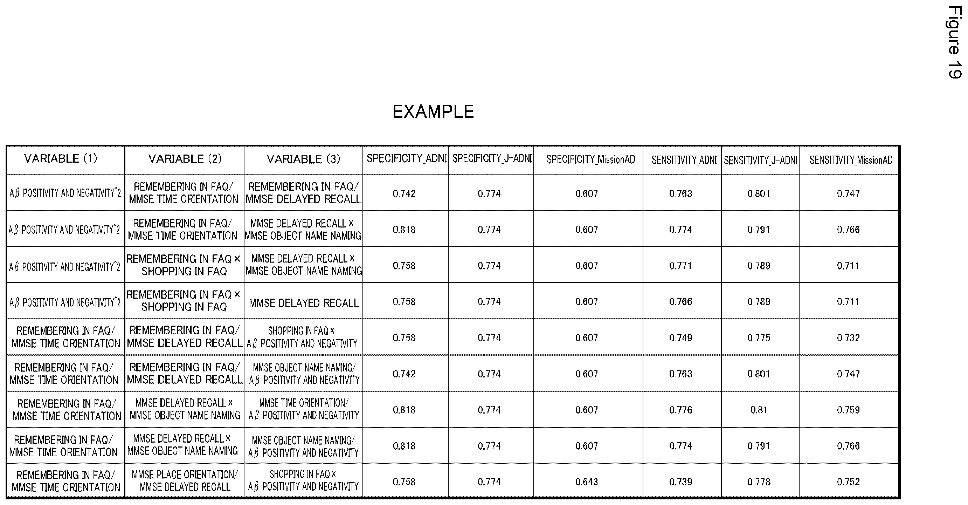

Figures 18 and 19 are diagrams showing examples of data used to evaluate the prediction models 132 of the example. Figure 18 shows an example of evaluation values for accuracy and sensitivity, which are examples of evaluation indices of the prediction models 132, using the evaluation data of ADNI, J-ADNI, and MissionAD. Figure 19 shows an example of evaluation values for specificity and AUC, which are examples of evaluation indices of the prediction models 132, using the evaluation data of ADNI, J-ADNI, and MissionAD. In the examples shown in Figures 18 and 19, in the data used to evaluate the prediction models 132, for example, the at least three variables include a first variable which is information obtained by squaring information on the positivity and negativity of amyloid β from among the fourth information, a second variable which includes, as a second feature amount, a feature amount obtained by dividing information on remembering holidays, family gatherings, appointments, or medication schedules in IADL evaluated by FAQ from among the third information by information on time orientation from among the first information, and a third variable which includes, as a second feature amount, a feature amount obtained by dividing information on remembering holidays, family gatherings, appointments, or medication schedules in IADL evaluated by FAQ from among the third information by information on delayed recall from among the second information. Furthermore, the at least three variables include, for example, a first variable that is information obtained by squaring information on the positivity and negativity of amyloid β from among the fourth information, a second variable that includes, as a second feature amount, a feature amount obtained by dividing information on remembering holidays, family gatherings, appointments, or medication schedules in IADL evaluated by FAQ from among the third information by information on time orientation from among the first information, and a third variable that includes, as a first feature amount, a feature amount obtained by integrating information on delayed recall from among the second information and information on object name naming from among the second information. In addition, the at least three variables include, for example, a first variable which is information obtained by squaring information on the positivity and negativity of amyloid β from among the fourth information, a second variable which includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, and a third variable which includes, as a first feature amount, a feature amount obtained by integrating information on delayed recall from among the second information and information on object name naming from among the second information. Furthermore, the at least three variables include, for example, a first variable which is information obtained by squaring information on the positivity and negativity of amyloid β from among the fourth information, a second variable which includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, and a third variable which is information on delayed recall from among the second information. In addition, the at least three variables include, for example, a first variable which includes, as a second feature amount, a feature amount obtained by dividing information on remembering holidays, family gatherings, appointments, or medication schedules in IADL evaluated by FAQ from among the third information by information on time orientation from among the first information, a second variable that includes, as a second feature amount, a feature amount obtained by dividing information on remembering holidays, family gatherings, appointments, or medication schedules in IADL evaluated by FAQ from among the third information by information on delayed recall from among the second information, and a third variable that includes, as a first feature amount, a feature amount obtained by integrating information on shopping in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a second feature amount, a feature amount obtained by dividing information on remembering holidays, family gatherings, appointments, or medication schedules in IADL evaluated by FAQ from among the third information by information on delayed recall from among the second information, and a third variable that includes, as a second feature amount, a feature amount obtained by dividing information on object name naming from among the second information by information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on delayed recall from among the second information and information on object name naming from among the second information, and a third variable that includes, as a second feature amount, a feature amount obtained by dividing information on time orientation from among the first information by information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on delayed recall from among the second information and information on object name naming from among the second information, and a third variable that includes, as a second feature amount, a feature amount obtained by dividing information on object name naming from among the second information by information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on place orientation from among the first information and information on delayed recall from among the second information, and a third variable that includes, as a first feature amount, a feature amount obtained by integrating information on shopping in IADL evaluated by FAQ from among the third information and information on the positivity and negativity of amyloid β from among the fourth information. In the above example, in evaluation of all of the prediction models 132 using the ADNI, J-ADNI, and MissionAD, sensitivity evaluation values are "0.7" or more, specificity evaluation values are "0.6" or more, and AUC evaluation values are "0.7" or more.

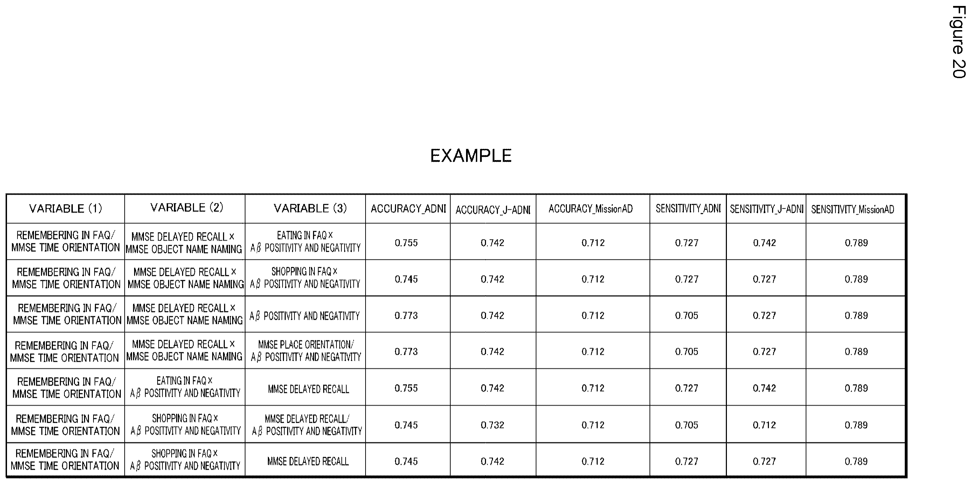

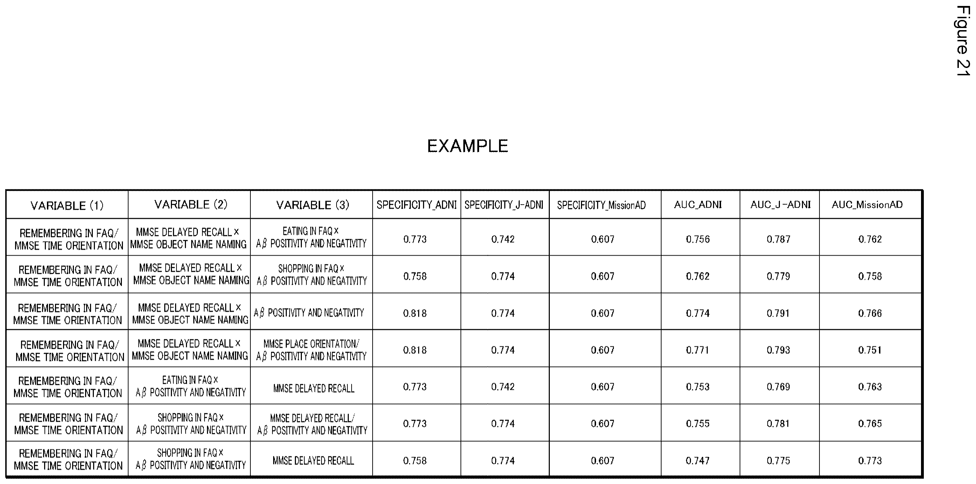

Figures 20 and 21 are diagrams showing examples of data used in evaluation of the prediction models 132 of the example. Figure 20 shows an example of evaluation values for accuracy and sensitivity, which are examples of evaluation indices of the prediction models 132, using the evaluation data of ADNI, J-ADNI, and MissionAD. Figure 21 shows an example of evaluation values for specificity and AUC, which are examples of evaluation indices of the prediction models 132, using the evaluation data of ADNI, J-ADNI, and MissionAD. In the examples shown in Figures 20 and 21, in the data used for evaluating the prediction model 132, for example, at least three variables are configured as a first variable that includes, as a second feature amount, a feature amount obtained by dividing information on remembering holidays, family gatherings, appointments, or medication schedules in IADL evaluated by FAQ among the third information by information on time orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on eating in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on shopping in IADL evaluated by FAQ from among the third information and information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on delayed recall from among the second information and information on object name naming from among the second information, and a third variable which is information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on delayed recall from among the second information and information on object name naming from among the second information, and a third variable that includes, as a second feature amount, a feature amount obtained by dividing place orientation from among the first information by information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on eating in IADL evaluated by FAQ from among the third information and information on the positivity and negativity of amyloid β from among the fourth information, and a third variable which is information on delayed recall from among the second information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on shopping in IADL evaluated by FAQ from among the third information and information on the positivity and negativity of amyloid β from among the fourth information, and a third variable that includes, as a second feature amount, a feature amount obtained by dividing information on delayed recall from among the second information by information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a first feature amount, a feature amount obtained by integrating information on shopping in IADL evaluated by FAQ from among the third information and information on the positivity and negativity of amyloid β from among the fourth information, and a third variable that is information on delayed recall from among the second information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

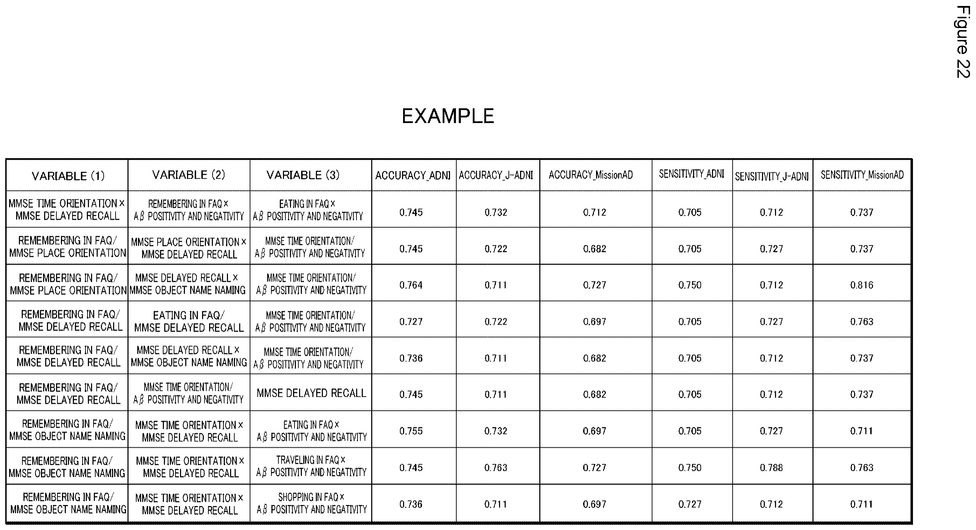

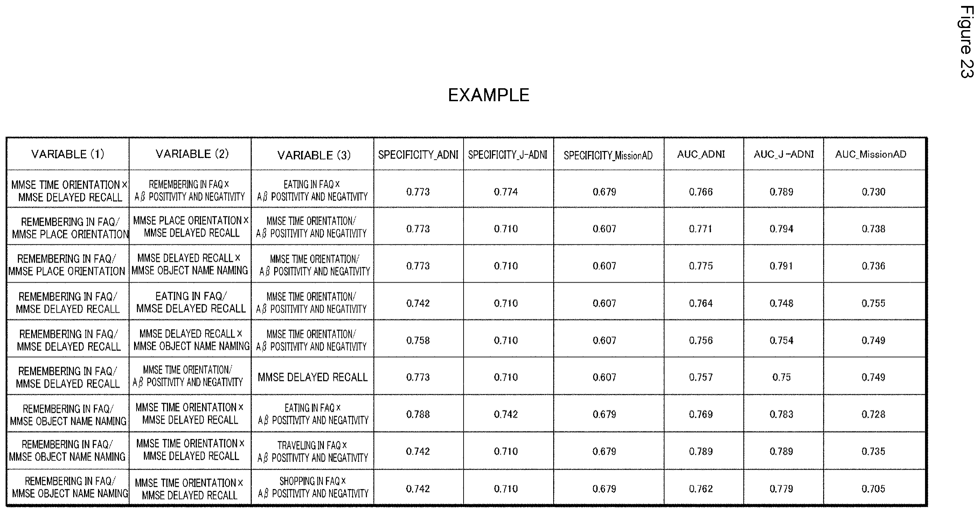

Figures 22 and 23 are diagrams showing examples of data used to evaluate the prediction models 132 of the example. Figure 22 shows an example of evaluation values for accuracy and sensitivity, which are examples of evaluation indices of the prediction models 132, using the evaluation data of ADNI, J-ADNI, and MissionAD. Figure 23 shows an example of evaluation values for specificity and AUC, which are examples of evaluation indices of the prediction models 132, using the evaluation data of ADNI, J-ADNI, and MissionAD. In the examples shown in Figures 22 and 23, in the data used to evaluate the prediction models 132, for example, the at least three variables include a first variable that includes, as a first feature amount, a feature amount obtained by integrating information on time orientation from among the first information and information on delayed recall from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that includes, as a first feature amount, a feature amount obtained by integrating information on eating in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on place orientation from among the first information, a second variable that includes, as a first feature amount, a feature amount obtained by integrating information on place orientation from among the first information and information on delayed recall from among the second information, and a third variable that includes, as a second feature amount, a feature amount obtained by dividing information on time orientation from among the first information by information on the positivity and negativity of amyloid β from among the fourth information. In addition, the at least three variables include, for example, a first variable that include, as a second feature amount, a feature amount obtained by dividing information on remembering holidays, family gatherings, appointments, or medication schedules in IADL evaluated by FAQ from among the third information by information on place orientation from among the first information, a second variable that includes, as a second feature amount, a feature amount obtained by integrating information on delayed recall from among the second information and information on object name naming from among the second information, and a third variable that includes, as a second feature amount, a feature amount obtained by dividing information on time orientation from among the first information by information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on delayed recall from among the second information, a second variable that includes, as a second feature amount, a feature amount obtained by dividing information on eating in IADL evaluated by FAQ from among the third information by information on delayed recall from among the second information, and a third variable that includes, as a second feature amount, a feature amount obtained by dividing information on time orientation from among the first information by information on the positivity and negativity of amyloid β from among the fourth information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on delayed recall from among the second information, a second variable that includes, as a first feature amount, a feature amount obtained by integrating information on delayed recall from among the second information and information on object name naming from among the second information, and a third variable that includes, as a second feature amount, a feature amount obtained by dividing information on time orientation from among the first information by information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on delayed recall from among the second information, a second variable that includes, as a second feature amount, a feature amount obtained by dividing information on time orientation from among the first information by information on the positivity and negativity of amyloid β from among the fourth information, and a third variable that is information on delayed recall from among the second information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on object name naming from among the second information, a second variable that includes, as a first feature amount, a feature amount obtained by integrating information on time orientation from among the first information and information on delayed recall from among the second information, and a third variable that includes, as a second feature amount, a feature amount obtained by integrating information on eating in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on remembering holidays, family gatherings, appointments, or medication schedules in IADL evaluated by FAQ from among the third information by information on object name naming from among the second information, a second variable that includes, as a first feature amount, a feature amount obtained by integrating information on time orientation from among the first information and information on delayed recall from among the second information, and a third variable that includes, as a first feature amount, a feature amount obtained by integrating information on travel in IADL evaluated by FAQ from among the third information and information on the positivity and negativity of amyloid β from among the fourth information. Moreover, the at least three variables include, for example, a first variable that includes, as a second feature amount, a feature amount obtained by dividing information on remembering holidays, family gatherings, appointments, or medication schedules in IADL evaluated by FAQ from among the third information by information on object name naming from among the second information, a second variable that includes, as a first feature amount, a feature amount obtained by integrating information on time orientation from among the first information and information on delayed recall from among the second information, and a third variable that includes, as a first feature amount, a feature amount obtained by integrating information on shopping in IADL evaluated by FAQ from among the third information and information on the positivity and negativity of amyloid β from among the fourth information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

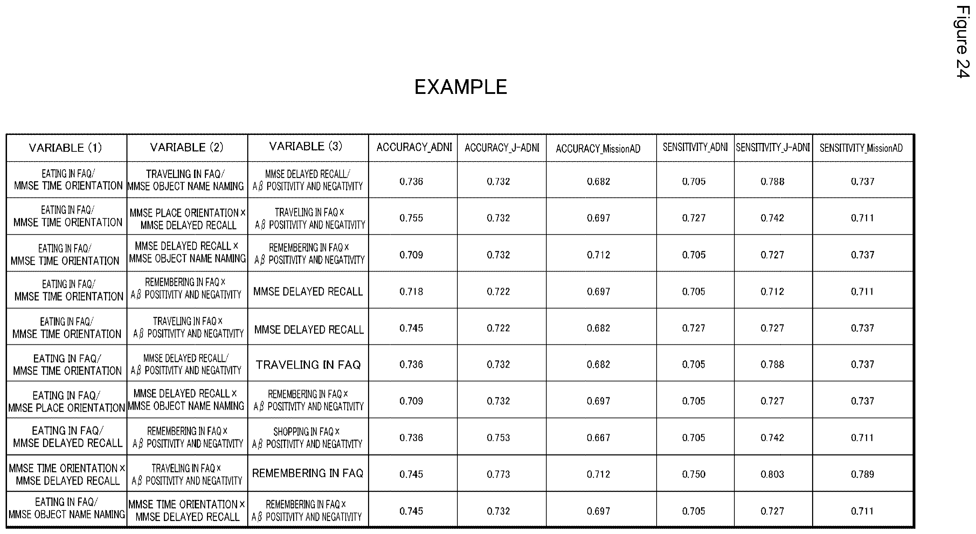

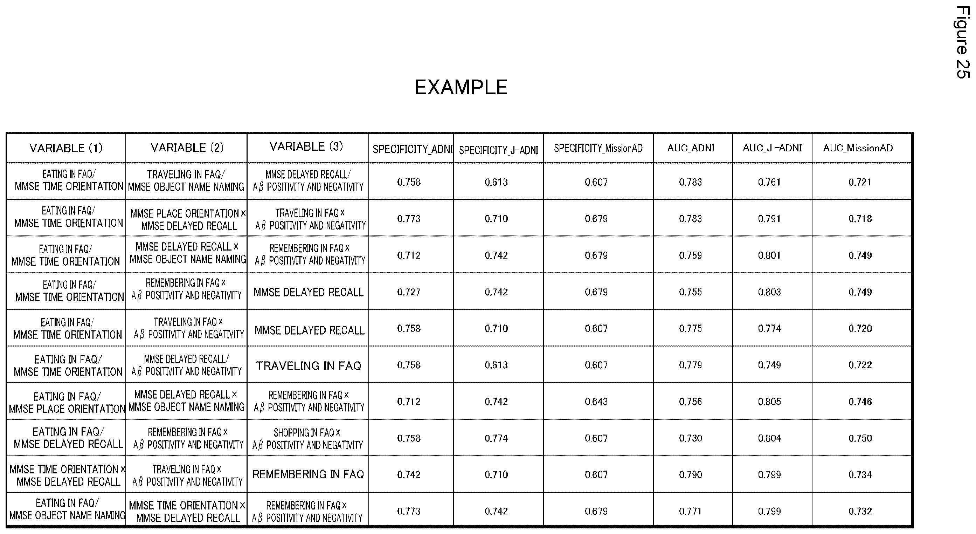

Figures 24 and 25 are diagrams showing example of data used to evaluate the prediction models 132 of the example. Figure 24 shows an example of evaluation values for accuracy and sensitivity, which are examples of evaluation indices of the prediction models 132, using the evaluation data of ADNI, J-ADNI, and MissionAD. Figure 25 shows an example of evaluation values for specificity and AUC, which are examples of evaluation indices of the prediction models 132, using the evaluation data of ADNI, J-ADNI, and MissionAD. In the examples shown in Figures 24 and 25, in the data used to evaluate the prediction models 132, for example, the at least three variables include a first variable that includes, as a second feature amount, a feature amount obtained by dividing information on remembering eating in IADL evaluated by FAQ from among the third information by information on time orientation from among the first information, a second variable that includes, as a second feature amount, a feature amount obtained by dividing information on travel in IADL evaluated by FAQ from among the third information by information on object name naming from among the second information, and a third variable that includes, as a second feature amount, a feature amount obtained by dividing information on delayed recall from among the second information by information on the positivity and negativity of amyloid β from among the fourth information. Furthermore, the at least three variables include, for example, a first variable that includes, as a second feature amount, a feature amount obtained by dividing information on eating in IADL evaluated by FAQ from among the third information by information on time orientation from among the first information, a second variable that includes, as a first feature amount, a feature amount obtained by integrating information on place orientation from among the first information and information on delayed recall from among the second information, and a third variable that includes, as a first feature amount, a feature amount obtained by integrating information on travel in IADL evaluated by FAQ from among the third information and information on the positivity and negativity of amyloid β from among the fourth information. In addition, the at least three variables include, for example, a first variable that includes, as a second feature amount, a feature amount obtained by dividing information on eating in IADL evaluated by FAQ from among the third information by information on time orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on eating in IADL evaluated by FAQ from among the third information by information on time orientation from among the first information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that is information on delayed recall from among the second information. In addition, the at least three variables include, for example, a first variable that includes, as a second feature amount, a feature amount obtained by dividing information on eating in IADL evaluated by FAQ from among the third information by information on time orientation from among the first information, a second variable that includes, as a first feature amount, a feature amount obtained by integrating information on travel in IADL evaluated by FAQ from among the third information and information on the positivity and negativity of amyloid β from among the fourth information, and a third variable which is information on delayed recall from among the second information. Furthermore, the at least three variables, for example, a first variable that includes, as a second feature amount, a feature amount obtained by dividing information on eating in IADL evaluated by FAQ from among the third information by information on time orientation from among the first information, a second variable that includes, as a second feature amount, a feature amount obtained by dividing information on delayed recall from among the second information by information on the positivity and negativity of amyloid β from among the fourth information, and a third variable which is information on travel in IADL evaluated by FAQ from among the third information. In addition, the at least three variables include, for example, a first variable that includes, as a second feature amount, a feature amount obtained by dividing information on eating in IADL evaluated by FAQ from among the third information by information on place orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. Furthermore, the at least three variables include, for example, a first variable that includes, as a second feature amount, a feature amount obtained by dividing information on eating in IADL evaluated by FAQ from among the third information by information on delayed recall from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that includes, as a first feature amount, a feature amount obtained by integrating information on shopping in IADL evaluated by FAQ from among the third information and information on the positivity and negativity of amyloid β from among the fourth information. In addition, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on travel in IADL evaluated by FAQ from among the third information and information on the positivity and negativity of amyloid β from among the fourth information, and a third variable which is information on remembering holidays, family gatherings, appointments, or medication schedules in IADL evaluated by FAQ from among the third information. Moreover, the at least three variables include, for example, a first variable that includes, as a second feature amount, a feature amount obtained by dividing information on eating in IADL evaluated by FAQ from among the third information by information on object name naming from among the second information, a second variable that includes, as a first feature amount, a feature amount obtained by integrating information on time orientation from among the first information and information on delayed recall from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. In the above example, in evaluation of all of the prediction models 132 using the ADNI, J-ADNI, and MissionAD, sensitivity evaluation values are "0.7" or more, specificity evaluation values are "0.6" or more, and AUC evaluation values are "0.7" or more.

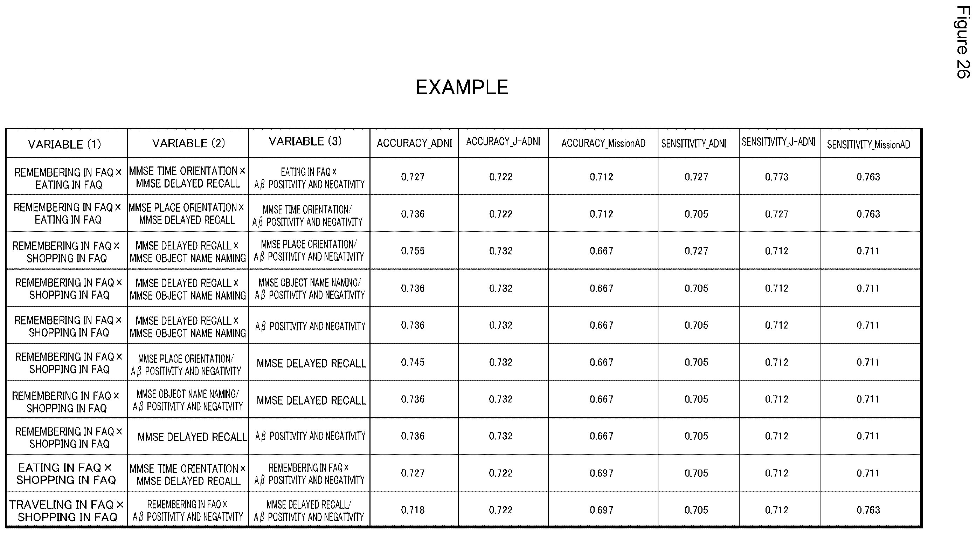

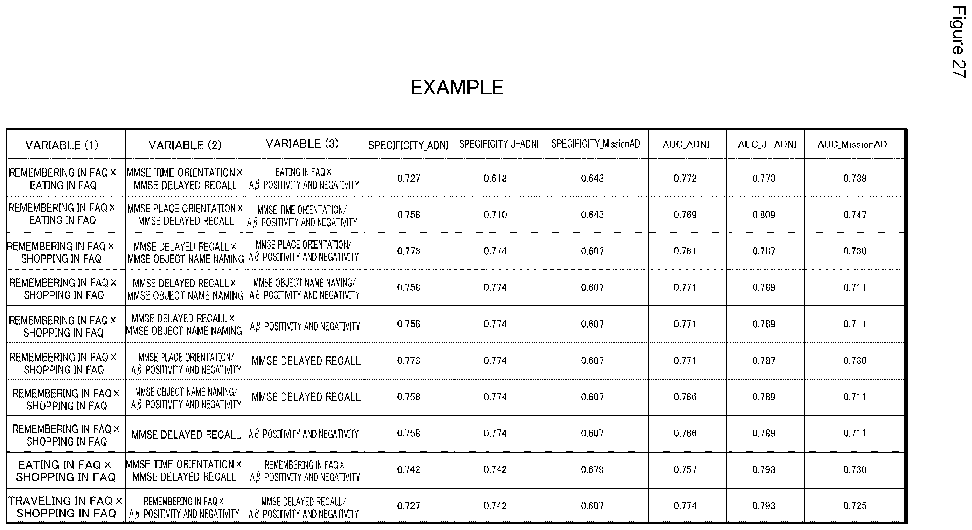

Figures 26 and 27 are diagrams showing examples of data used to evaluate the prediction models 132 of the example. Figure 26 shows an example of evaluation values for accuracy and sensitivity, which are examples of evaluation indices of the prediction models 132, using the evaluation data of ADNI, J-ADNI, and MissionAD. Figure 27 shows an example of evaluation values for specificity and AUC, which are examples of evaluation indices of the prediction models 132, using the evaluation data of ADNI, J-ADNI, and MissionAD. In the examples shown in Figures 26 and 27, in the data used to evaluate the prediction models 132, for example, the at least three variables include a first variable that includes, as a first feature amount, a feature amount obtained by integrating information on remembering holidays, family gatherings, appointments, or medication schedules in IADL evaluated by FAQ from among the third information and information on eating in IADL evaluated by FAQ from among the third information, a second variable that includes, as a first feature amount, a feature amount obtained by integrating information on time orientation from among the first information and information on delayed recall from among the second information, and a third variable that includes, as a first feature amount, a feature amount obtained by integrating information on eating in IADL evaluated by FAQ from among the third information and information on the positivity and negativity of amyloid β from among the fourth information. Furthermore, the at least three variables include, for example, a first variable that includes, as a first feature amount, a feature amount obtained by integrating information on remembering holidays, family gatherings, appointments, or medication schedules in IADL evaluated by FAQ from among the third information and information on eating in IADL evaluated by FAQ from among the third information, a second variable that includes, as a first feature amount, a feature amount obtained by integrating information on place orientation from among the first information and information on delayed recall from among the second information, and a third variable that includes, as a second feature amount, a feature amount obtained by dividing information on time orientation from among the first information by information on the positivity and negativity of amyloid β from among the fourth information. In addition, the at least three variables include, for example, a first variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, a second variable that includes, as a first feature amount, a feature amount obtained by integrating information on delayed recall from among the second information and information on object name naming from among the second information, and a third variable that includes, as a second feature amount, a feature amount obtained by dividing information on place orientation from among the first information by information on the positivity and negativity of amyloid β from among the fourth information. Furthermore, the at least three variables include, for example, a first variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, a second variable that includes, as a first feature amount, a feature amount obtained by integrating information on delayed recall from among the second information and information on object name naming from among the second information, and a third variable that includes, as a second feature amount, a feature amount obtained by dividing information on object name naming from among the second information by information on the positivity and negativity of amyloid β from among the fourth information. In addition, the at least three variables include, for example, a first variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, a second variable that includes, as a first feature amount, a feature amount obtained by integrating information on delayed recall from among the second information and information on object name naming from among the second information, and a third variable that is information on the positivity and negativity of amyloid β from among the fourth information. In addition, the at least three variables include, for example, a first variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, a second variable that includes, as a second feature amount, a feature amount obtained by dividing information on place orientation from among the first information by information on the positivity and negativity of amyloid β from among the fourth information, and a third variable that is information on delayed recall from among the second information. In addition, the at least three variables include, for example, a first variable that includes, as a first feature amount, a feature amount obtained by integrating information on remembering holidays, family gatherings, appointments, or medication schedules in IADL evaluated by FAQ among the third information and information on shopping in IADL evaluated by FAQ from among the third information, a second variable that includes, as a second feature amount, a feature amount obtained by dividing information on object name naming from among the second information by information on the positivity and negativity of amyloid β from among the fourth information, and a third variable that is information on delayed recall from among the second information. Furthermore, the at least three variables include, for example, a first variable that includes, as a first feature amount, a feature amount obtained by integrating information on remembering holidays, family gatherings, appointments, or medication schedules in IADL evaluated by FAQ from among the third information and information on shopping in IADL evaluated by FAQ from among the third information, a second variable that is information on delayed recall from among the second information, and a third variable that is information on the positivity and negativity of amyloid β from among the fourth information. In addition, the at least three variables include, for example, a first variable that includes, as a first feature amount, a feature amount obtained by integrating information on eating in IADL evaluated by FAQ from among the third information and information on shopping in IADL evaluated by FAQ from among the third information, a second variable that includes, as a first feature amount, a feature amount obtained by integrating information on time orientation from among the first information and information on delayed recall from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. Moreover, the at least three variables include, for example, a first variable that includes, as a first feature amount, a feature amount obtained by integrating information on travel in IADL evaluated by FAQ from among the third information and information on shopping in IADL evaluated by FAQ from among the third information, a second variable that includes, as a first feature amount, a feature amount obtained by integrating information on remembering holidays, family gatherings, appointments, or medication schedules in IADL evaluated by FAQ from among the third information and information on positivity and negativity of amyloid β from among the fourth information, and a third variable that includes, as a second feature amount, a feature amount obtained by dividing information on delayed recall from among the second information by information on the positivity and negativity of amyloid β from among the fourth information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

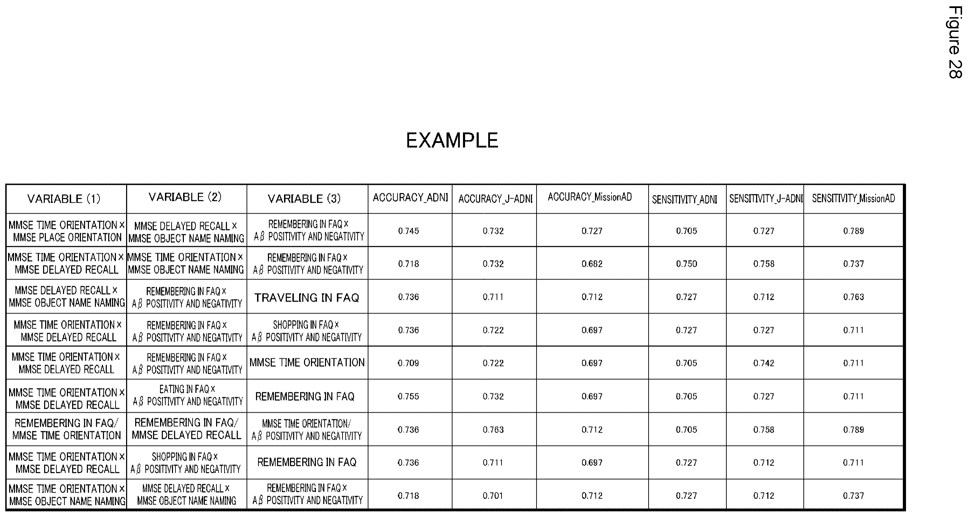

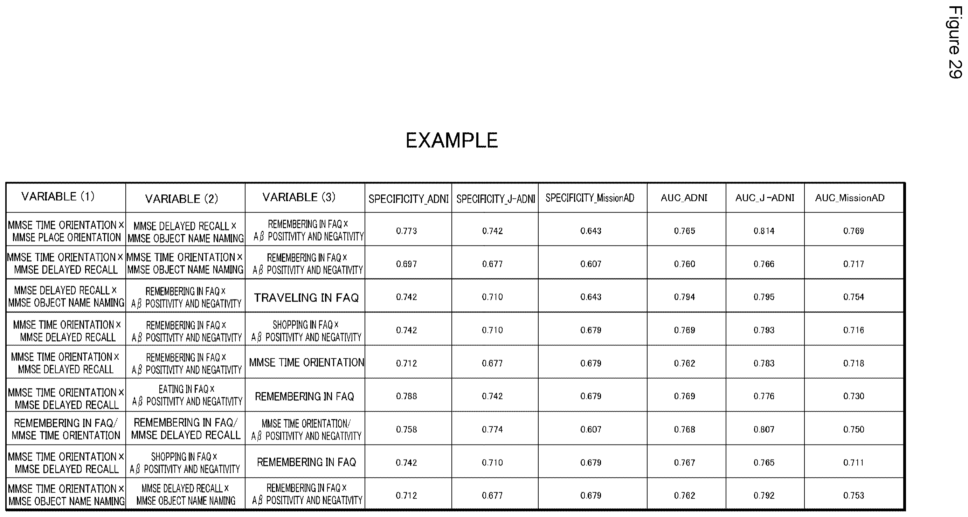

Figures 28 and 29 are diagrams showing examples of data used to evaluate the prediction models 132 of the example. Figure 28 shows an example of evaluation values for accuracy and sensitivity, which are examples of evaluation indices of the prediction models 132, using the evaluation data of ADNI, J-ADNI, and MissionAD. Figure 29 shows an example of evaluation values for specificity and AUC, which are examples of evaluation indices of the prediction models 132, using the evaluation data of ADNI, J-ADNI, and MissionAD. In the examples shown in Figures 28 and 29, in the data used to evaluate the prediction models 132, for example, the at least three variables include a first variable that includes, as a first feature, a feature amount obtained by integrating information on time orientation from among the first information and information on place orientation from among the first information, a second variable that includes, as a first feature amount, a feature amount obtained by integrating information on delayed recall from among the second information and information on object name naming from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. Furthermore, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on time orientation from among the first information and information on object name naming from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. In addition, the at least three variables include, for example, a first variable that includes, as a first feature amount, a feature amount obtained by integrating information on delayed recall from among the second information and information on object name naming from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that is information on travel in IADL evaluated by FAQ from among the third information. Furthermore, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that includes, as a first feature amount, a feature amount obtained by integrating information on shopping in IADL evaluated by FAQ from among the third information and information on the positivity and negativity of amyloid β from among the fourth information. In addition, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that is information on time orientation from among the first information. Furthermore, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on eating in IADL evaluated by FAQ from among the third information and information on the positivity and negativity of amyloid β from among the fourth information, and a third variable that is information on remembering holidays, family gatherings, appointments, or medication schedules in IADL evaluated by FAQ from among the third information. In addition, the at least three variables include, for example, a first variable that includes, as a second feature amount, a feature amount obtained by dividing information on remembering holidays, family gatherings, appointments, or medication schedules in IADL evaluated by FAQ from among the third information by information on time orientation from among the first information, a second variable that includes, as a second feature amount, a feature amount obtained by dividing information on remembering holidays, family gatherings, appointments, or medication schedules in IADL evaluated by FAQ from among the third information by information on delayed recall from among the second information, and a third variable that includes, as a second feature amount, a feature amount obtained by dividing information on time orientation from among the first information by information on the positivity and negativity of amyloid β from among the fourth information. Furthermore, the at least three variables include, for example, a first variable that includes, as a first feature amount, a feature amount obtained by integrating information on time orientation from among the first information and information on delayed recall from among the second information, a second variable that includes, as a first feature amount, a feature amount obtained by integrating information on shopping in IADL evaluated by FAQ from among the third information and information on the positivity and negativity of amyloid β from among the fourth information, and a third variable that is information on remembering holidays, family gatherings, appointments, or medication schedules in IADL evaluated by FAQ from among the third information. Moreover, the at least three variables include, for example, a first variable that includes, as a first feature amount, a feature amount obtained by integrating information on time orientation from among the first information and information on object name naming from among the second information, a second variable that includes, as a first feature amount, a feature amount obtained by integrating information on delayed recall from among the second information and information on object name naming from among the second information, and a third variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

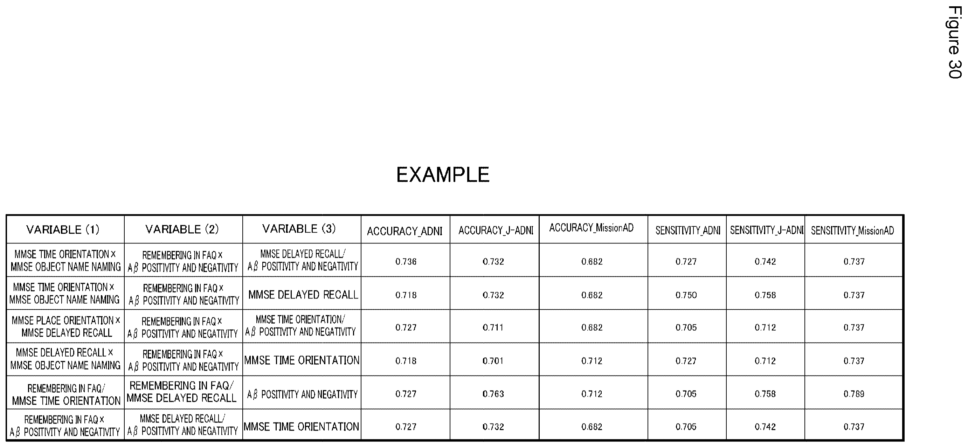

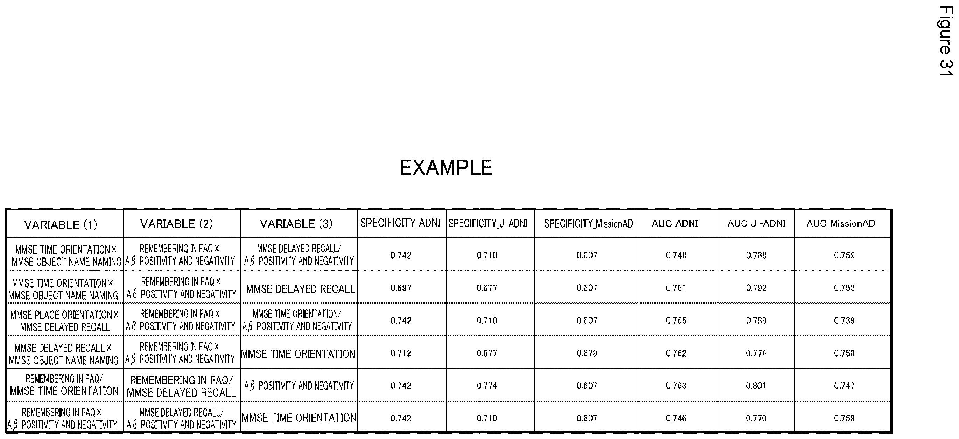

Figures 30 and 31 are diagrams showing examples of data used to evaluate the prediction models 132 of the example. Figure 30 shows an example of evaluation values for accuracy and sensitivity, which are examples of evaluation indices of the prediction models 132, using the evaluation data of ADNI, J-ADNI, and MissionAD. Figure 31 shows an example of evaluation values for specificity and AUC, which are examples of evaluation indices of the prediction models 132, using the evaluation data of ADNI, J-ADNI, and MissionAD. In the examples shown in Figures 30 and 31, in the data used to evaluate the prediction models 132, for example, the at least three variables include a first variable that includes, as a first feature amount, a feature amount obtained by integrating information on time orientation from among the first information and information on object name naming from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that includes, as a second feature amount, a feature amount obtained by dividing information on delayed recall from among the second information by information on the positivity and negativity of amyloid β from among the fourth information. Furthermore, the at least three variables include, for example, a first variable that includes, as a first feature amount, a feature amount obtained by integrating information on time orientation from among the first information and information on object name naming from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that is information on delayed recall from among the second information. In addition, the at least three variables include a first variable that includes, as a first feature amount, a feature amount obtained by integrating information on place orientation from among the first information and information on delayed recall from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that includes, as a second feature amount, a feature amount obtained by dividing information on time orientation from among the first information by information on the positivity and negativity of amyloid β from among the fourth information. Furthermore, the at least three variables include, for example, a first variable that includes, as a first feature amount, a feature amount obtained by integrating information on delayed recall from among the second information and information on object name naming from among the second information, a second variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, and a third variable that is information on time orientation from among the first information. In addition, the at least three variables include, for example, a first variable that includes, as a first feature amount, a feature amount obtained by dividing information on remembering holidays, family gatherings, appointments, or medication schedules in IADL evaluated by FAQ from among the third information by information on time orientation from among the first information, a second variable that includes, as a second feature amount, a feature amount obtained by dividing information on remembering holidays, family gatherings, appointments, or medication schedules in IADL evaluated by FAQ from among the third information by information on delayed recall from among the second information, and a third variable that is information on the positivity and negativity of amyloid β from among the fourth information. In addition, the at least three variables include, for example, a first variable that includes, as a first feature amount, a feature amount obtained by integrating information on remembering holidays, family gatherings, appointments, or medication schedules in IADL evaluated by FAQ from among the third information and information on the positivity and negativity of amyloid β from among the fourth information, a second variable that includes, as a first feature amount, a feature amount obtained by dividing information on delayed recall from among the second information by information on the positivity and negativity of amyloid β from among the fourth information, and a third variable that is information on time orientation from among the first information. In the above example, in evaluation of all of the prediction models 132 using ADNI, J-ADNI, and MissionAD, sensitivity evaluation values are "0.7" or more, specificity evaluation values are "0.6" or more, and AUC evaluation values are "0.7" or more.

According to the information processing device 100 described above, it is possible to achieve high prediction accuracy regardless of race, culture, customs, etc. by selecting, as training data used to train the prediction models 132, data that is highly evaluated in evaluation based on a plurality of pieces of evaluation data 131, such as ADNI, J-ADNI, and MissionAD, which are not easily influenced by differences in culture, customs, and preferences because they cover a wide area. Further, it is possible to shorten and reduce the consultation (questionnaire) time, the physical burden of examination (biological sample collection), and the number of visits to the hospital, and to reduce the processing load on patients and devices by selecting, as training data used to train the prediction models 132, data that can be relatively easily obtained by questionnaires to patients.

### [Hardware Configuration]

Figure 31 is a diagram showing an example of a hardware configuration of the information processing device 100 of the present embodiment. As shown in the figure, the information processing device 100 is configured such that a communication controller 100-1, a CPU 100-2, a random access memory (RAM) 100-3 used as a working memory, a read only memory (ROM) 100-4 in which a boot program and the like are stored, a storage device 100-5 such as a flash memory or a hard disk drive (HDD), a drive device 100-6, etc. are connected to each other through an internal bus or a dedicated communication line. The communication controller 100-1 communicates with components other than the information processing device 100. The storage device 100-5 stores a program 100-5a executed by the CPU 100-2. This program is deployed in the RAM 100-3 by a direct memory access (DMA) controller (not shown) or the like, and executed by the CPU 100-2. Accordingly, the acquisition unit 121, the prediction unit 122, and the output unit 123 are implemented.

The above-described embodiment is intended to facilitate understanding of the present invention, and is not intended to limit the interpretation of the present invention. The present invention may be modified or improved without departing from the spirit of the present invention, and equivalents thereof are also included in the present invention. That is, even those in which a person skilled in the art appropriately modifies the design of each embodiment are included within the scope of the present invention as long as they include the features of the present invention. In addition, each embodiment is an example, and it goes without saying that partial replacement or combination of the configurations shown in different embodiments is possible, and these are also included within the scope of the present invention as long as they include the features of the present invention.

### Reference Signs List

- 10: Terminal device
- 100: Information processing device
- 110: Communication device
- 120: Controller
- 121: Acquisition unit
- 122: Prediction unit
- 123: Output unit
- 130: Storage
- 131: Evaluation data
- 132: Prediction model
- NW: Communication network

## Claims

1. An information processing device,
wherein patient information corresponding to each of at least three variables is input to a prediction model trained using training data in which the three variables are associated with presence or absence of progression of symptoms of mild cognitive impairment (MCI) or mild Alzheimer's dementia (mild AD) to predict a probability of progression of a patient's symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers,
wherein, with respect to at least four pieces of information, selected from the first information, the second information, the third information, and the fourth information and including: at least one of information on orientation relating to time from among the first information and information on remembering a holiday, a family gathering, an appointment, or a medication schedule in IADL from among the third information; information on delayed recall from among the second information; and information on positivity and negativity of amyloid β from among the fourth information, the at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

2. The information processing device according to claim 1, wherein the at least three variables include at least one of the first feature amount and the second feature amount with respect to at least four pieces of information including: information on remembering a holiday, a family gathering, an appointment, or a medication schedule in IADL from among the third information; information on delayed recall from among the second information; and the fourth information.

3. The information processing device according to claim 1, wherein the at least three variables include at least one of the first feature amount and the second feature amount with respect to at least four pieces of information including: information on orientation relating to time from among the first information; information on remembering a holiday, a family gathering, an appointment, or a medication schedule in IADL from among the third information; information on delayed recall from among the second information; and the fourth information.

4. The information processing device according to claim 3, wherein the at least three variables include at least one of the first feature amount and the second feature amount with respect to four or five pieces of information.

5. The information processing device according to claim 3, wherein the at least three variables include at least one of the first feature amount and the second feature amount with respect to four pieces of information including: information on orientation relating to time from among the first information; information on remembering a holiday, a family gathering, an appointment, or a medication schedule in IADL from among the third information; information on delayed recall from among the second information; and the fourth information.

6. The information processing device according to claim 1, wherein the at least three variables include at least one of the first feature amount and the second feature amount with respect to five pieces of information including: information on orientation relating to time from among the first information; information on remembering a holiday, a family gathering, an appointment, or a medication schedule in IADL from among the third information; information on delayed recall from among the second information; the fourth information; and fifth information selected from the first information, the second information, the third information, and the fourth information.

7. An information providing method comprising the steps of:
acquiring patient information;
predicting a probability of progression of patient's symptoms of mild cognitive impairment (MCI) or mild Alzheimer's dementia (mild AD) by inputting patient information corresponding to each of at least three variables to a prediction model trained using training data in which the three variables are associated with presence or absence of progression of symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers; and
outputting a prediction result on the probability of progression of a patient's symptoms of mild cognitive impairment (MCI) or mild Alzheimer's dementia (mild AD),
wherein, with respect to at least four pieces of information, selected from the first information, the second information, the third information, and the fourth information and including: at least one of information on orientation relating to time from among the first information and information on remembering a holiday, a family gathering, an appointment, or a medication schedule in IADL from among the third information; information on delayed recall from among the second information; and information on positivity and negativity of amyloid β from among the fourth information, the at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

8. A program causing a computer to execute:
processing of acquiring patient information;
processing of predicting a probability of progression of a patient's symptoms of mild cognitive impairment (MCI) or mild Alzheimer's dementia (mild AD) by inputting patient information corresponding to each of at least three variables to a prediction model trained using training data in which the three variables are associated with presence or absence of progression of symptoms of mild cognitive impairment (MCI) or mild Alzheimer's dementia (mild AD), the at least three variables being based on information selected from among first information on orientation, second information on remembering, third information on remembering a holiday, a family gathering, an appointment, or a medication schedule in IADL or an actual action of eating, shopping, or traveling in IADL, and fourth information on positivity and negativity biomarkers; and
processing of outputting a prediction result on the probability of progression of patient's symptoms of mild cognitive impairment (MCI) or mild Alzheimer's dementia (mild AD),
wherein, with respect to at least four pieces of information, selected from the first information, the second information, the third information, and the fourth information and including: at least one of information on orientation relating to time from among the first information and information on remembering a holiday, a family gathering, an appointment, or a medication schedule in IADL from among the third information; information on delayed recall from among the second information; and information on positivity and negativity of amyloid β from among the fourth information, the at least three variables include at least one of a first feature amount obtained by integrating information in which evaluation values indicating degrees of symptoms of mild cognitive impairment (MCI) or mild Alzheimer's dementia (mild AD) show a positive correlation, and a second feature amount obtained by dividing information in which magnitudes of evaluation values indicating degrees of symptoms of mild cognitive impairment (MCI) or mild Alzheimer's dementia (mild AD) show a negative correlation.

9. A non-transitory computer-readable storage medium storing the program according to claim 8.
